# EUROPEAN PATENT SPECIFICATION

(11) **EP 0 935 607 B1**
(45) Date of publication and mention of the grant of the patent: **28.07.2004**
(21) Application number: 97938386.6
(22) Date of filing: 15.08.1997
(51) Int. Cl.: C07K 14/00

(54) **SOLUBLE MONOVALENT AND MULTIVALENT MHC CLASS II FUSION PROTEINS, AND USES THEREFOR**
LÖSLICHE MONOVALENTE UND MULTIVALENTE MHC KLASSE II FUSIONSPROTEINE UND DEREN VERWENDUNGEN
PROTEINES DE FUSION DE CLASSE II DU CMH, SOLUBLES, MONOVALENTES OU POLYVALENTES, ET UTILISATIONS ASSOCIEES

(30) Priority: 16.08.1996 US 24077 P
(43) Date of publication of application: 18.08.1999
(73) Proprietor: THE PRESIDENT AND FELLOWS OF HARVARD COLLEGE, Cambridge, MA 02138-5701 (US)
(72) Inventor: WUCHERPFENNIG, Kai, W., Brookline, MA 02146 (US); STROMINGER, Jack, L., Lexington, MA 02173 (US)
(74) Representative: Jump, Timothy John Simon
(86) International application number: PCT/US1997/014503
(87) International publication number: WO 1998/006749

(56) References cited:
- WO-A-93/10220
- WO-A-96/04314
- COSSON, P. & BONIFACINO, J.S.: "Role of transmembrane domain interactions in the assembly of class II MHC molecules" SCIENCE, vol. 258, 1992, pages 659-662, XP002053286 cited in the application
- KOZONO, H. ET AL.: "Production of soluble MHC class II proteins with covalently bound single peptides" NATURE, vol. 369, 1994, pages 151-154, XP002036522
- CHANG, H.-C. ET AL.: "A general method for facilitating heterodimeric pairing ..." PROC. NATL. ACAD. SCI. USA, vol. 91, 1994, pages 11408-11412, XP002053287 cited in the application
- KALANDADZE, A. ET AL.: "Expression of recombinant HLA-DR2 molecules" J. BIOL. CHEM., vol. 271, 16 August 1996, pages 20156-20162, XP002053288

## Description

### Field of the Invention

The present invention is directed to the field of immunology. In particular, the present invention is directed to the design, production, and use of recombinant fusion proteins derived, in part, from the proteins of the human Major Histocompatibility Complex.

### Background of the Invention

MHC molecules are highly polymorphic dimeric proteins which determine the specificity of T cell mediated immune responses by binding peptides from foreign antigens in an intracellular processing compartment, and by presenting these peptides on the surface of antigen presenting cells, where they may be recognized by specialized T cell receptors (TCRs) (reviewed in Strominger and Wiley, 1995). For example, the MHC Class II DR β chain gene, with 137 known DRB 1 alleles (Marsh and Bodmer, 1995), is the most polymorphic human gene that has been identified. Not surprisingly, the polymorphic residues of these proteins are clustered in peptide binding domains which define the large repertoire of peptides that may be presented to T cells (Bjorkman et al., 1987; Stem et al., 1994). Although T cells should not normally react to self peptides presented in syngeneic MHC molecules, some alleles of the MHC Class II genes are believed to confer susceptibility to autoimmune diseases through the presentation of pathogenic self-peptides. Thus, for example, HLA-DR2 subtypes confer an increased risk for multiple sclerosis (MS), while subtypes of HLA-DR4 confer susceptibility to rheumatoid arthritis (reviewed in Todd et al., 1988; Wucherpfennig and Strominger, 1995b).

The production of soluble, "empty" MHC Class II molecules (i.e., molecules which do not have peptides bound within the MHC Class II peptide binding domains) would be highly useful in producing homogeneous preparations of MHC/peptide complexes "loaded" with a single variety of peptide. Such soluble, MHC/peptide complexes have several important investigational and therapeutic uses. For example, soluble MHC Class II molecules are required for crystallographic studies of single MHC/peptide complexes, and for studying the biochemical interaction of particular MHC/peptide complexes with their cognate TCRs. Structural characterization of the MHC/peptide/TCR recognition unit will provide important insights into the mechanisms by which MHC molecules confer susceptibility to autoimmunity. In addition, soluble MHC/peptide complexes are useful for the treatment of autoimmune diseases. For example, studies in the murine experimental autoimmune encephalomyelitis (EAE) model have demonstrated that an autoimmune disease can be treated by the administration of soluble MHC/peptide complexes loaded with the autoantigenic peptide (Sharma et al., 1991). Such complexes are expected to be useful in the treatment of several human autoimmune diseases, including multiple sclerosis (MS) and rheumatoid arthritis (RA).

A number of approaches have been followed to obtain purified, soluble, empty MHC Class II molecules. For example, MHC Class II molecules can be purified from mammalian cells by affinity chromatography following detergent solubilization of B cell membranes (Gorga et al., 1987). MHC molecules purified from B cell lines, however, have already passed through the intracellular MHC Class II peptide loading compartment and, therefore, are already loaded with a diverse set of peptides (Chicz et al., 1992). Furthermore, removal of these peptides from B cell derived MHC complexes (e.g., by low pH treatment) is very difficult and typically results in MHC protein denaturation. In another approach, soluble, truncated HLA-DR1 and HLA-DR4 molecules have been expressed in the baculovirus/insect cell system using cDNA constructs for the DRα and DRβ extracellular domains without the hydrophobic transmembrane domains (Stem and Wiley, 1992). These molecules were assembled and secreted but had a tendency to aggregate unless they were loaded with a high affinity peptide. Unfortunately, this approach has not been successful with HLA-DR2 molecules. For example, the product of the DRA, DRB5*0101 genes showed a strong tendency to aggregate even when high affinity peptides were added (Vranovsky and Strominger, unpublished observations). In addition, when this approach was attempted with the DR2 molecules formed by the DRA and DRB1*1501 gene products, the DRα and DRβ chains failed to assemble (Wucherpfennig, unpublished observations). In yet another approach, Wettstein et al. (1991) expressed a murine Class II heterodimer (E^{k}) as a glycan-phosphatidylinositol linked chimera which could be cleaved from CHO cells by phospholipase C to yield a soluble form, but this form required 100-fold higher concentrations of peptide to yield two- to four-fold lower levels of T cell stimulation. The expression of soluble mouse I-A molecules (I-A^{u} and I-A^{g7}, which confer susceptibility to EAE and diabetes, respectively) has also been difficult. When the extracellular domains of these MHC molecules were fused with a glycan-phosphatidyl inositol anchor and then cleaved from the surface of transfected cells, irreversible aggregation occurred even if the cells had been incubated with I-A binding peptides prior to cleavage (L. Fugger and H. McDevitt, personal communication). All of these observations with truncated MHC molecules suggest that, for some but not all of these proteins, the α-helical transmembrane regions of the MHC Class II α and β chains are essential to the normal assembly of the αβ heterodimer (Cosson and Bonifacino, 1992).

It has been suggested that "dimerization domains" of known, stable dimeric proteins may be genetically engineered into fusion proteins to promote the formation of stable dimeric fusion proteins. For example, synthetic peptides of the isolated Fos and Jun leucine zipper dimerization domains, with added N-terminal cysteine residues and (Gly)₂ linkers, were shown to assemble as soluble heterodimers with interchain disulfide bridges (O'Shea et al., 1989). Fusion proteins including artificial leucine zipper dimerization domains were also employed to express αβ heterodimers of the TCR extracellular domains with interchain disulfide bridges (Chang et al., 1994). Although these TCR chimeras were bound by antibodies to native TCRs, they were not shown to retain MHC/peptide complex specificity. In another approach, Grégiore et al. (1991) produced soluble αβ heterodimers of TCR extracellular domains by co-expressing proteins in which the variable and constant (first exon only) domains of α and β TCR chains were each fused to the same constant domain of an immunoglobulin κ light chain. Again, although the fusion heterodimers were recognized by antibodies to the native TCR, these authors were unable to measure direct binding of the fusion heterodimer to its cognate MHC antigen, and found that the fusion heterodimer failed to reproducibly inhibit T cells bearing the native TCR from recognizing cells bearing the cognate MHC antigen. Finally, Weber et al. (1992), using a similar approach, failed to detect direct MHC binding of a TCR fusion heterodimer but inferred low affinity binding from binding competition experiments.

### Summary of the Invention

The present invention is directed to monovalent and multivalent fusion proteins comprising human Class II Major Histocompatibility Complex binding domains, with or with bound MHC binding peptides, which are useful in diagnostic and therapeutic methods, as well as laboratory assays.

In one aspect, the present invention provides a soluble Class II Major Histocompatibility Complex fusion protein comprising:
a first polypeptide comprising a fusion of, toward the N-terminus, at least an MHC Class II binding domain of a MHC Class II α chain and, toward the C-terminus, a first coiled-coil dimerization domain; and
a second polypeptide comprising a fusion of, toward the N-terminus, at least an MHC Class II binding domain of a MHC Class II β chain and, toward the C-terminus, a second coiled-coil dimerization domain;
wherein said first and second polypeptides associate under physiological conditions to form a heterodimeric MHC Class II molecule that is capable of selectively binding an MHC binding peptide to form a MHC/peptide complex.

In one aspect, the present invention provides fusion proteins of MHC Class II α and β chain proteins in which substantially all of the C-terminal transmembrane and cytoplasmic domains have been replaced by dimerization domains and, optionally, interposing linker sequences.

Thus, a Class II MHC fusion protein is provided comprising a fusion of, toward the N-tenninus, at least an MHC Class II binding domain of an MHC Class II α chain and, toward the C-terminus, a dimerization domain. In preferred embodiments, the MHC Class II binding domain comprises an extracellular domain of an MHC Class II alpha chain, preferably at least residues 5-180 of an MHC Class II alpha chain, more preferably residues 5-190, and most preferably residues 5-200. The MHC Class II alpha chains from which the fusion proteins of the invention may be derived include HLA-DR1, HLA-DR2, HLA-DR4, HLA-DQ1, HLA-DQ2 and HLA-DQ8 alpha chains, and particularly alpha chains encoded by DRA*0101, DRA*0102, DQA1*0301 or DQA1*0501 alleles.

Similarly, a Class II MHC fusion protein is provided comprising a fusion of, toward the N-terminus, at least an MHC Class II binding domain of an MHC Class II β chain and, toward the C-terminus, a dimerization domain. In preferred embodiments, the MHC Class II binding domain comprises an extracellular domain of an MHC Class II beta chain, preferably at least residues 5-185 of an MHC Class 11 alpha chain, more preferably residues 5-195, and most preferably residues 5-205. The MHC Class II alpha chains from which the fusion proteins of the invention may be derived include HLA-DR1, HLA-DR2, HLA-DR4, HLA-DQ1, HLA-DQ2 and HLA-DQ8 beta chains, and particularly beta chains encoded by DRB1*01, DRB1*15, DRB1*16, DRB5*01, DQB1*03 and DQB1*02 alleles.

In some preferred embodiments, the dimerization domains of the Class II MHC fusion proteins comprise coiled-coil dimerization domains, such as leucine zipper domains. Preferably, the leucine zipper domains include at least four leucine heptads. In one preferred embodiment, the leucine zipper domain is a Fos or Jun leucine zipper domain.

In other embodiments which do not form part of the claimed invention, the dimenzation domain is an immunoglobulin Fab constant domain, such as an immunoglobulin heavy chain C_{H}1 constant region or an immunoglobulin light chain constant region.

In each of the foregoing embodiments, a flexible molecular linker optionally may be interposed between, and covalently join, the MHC Class II chain and the dimerization domain. Preferably, the flexible molecular linker comprises a peptide sequence of 1-15 amino acid residues, more preferably 5-7 amino acid residues. In addition, when polypeptide linkers are employed, it is preferred that a majority of the amino acid residues in the linker are alanine, glycine, serine, leucine, isoleucine, or valine residues.

In addition, in each of the foregoing embodiments, an MHC Class II binding peptide optionally may be covalently joined to the N-terminus of the MHC Class II alpha or beta chain, such that the binding peptide is capable of selectively binding to an MHC Class II molecule formed by the alpha or beta chain and another (beta or alpha, respectively) MHC Class II chain. Thus, the MHC binding peptide and the MHC Class II chains form an MHC/peptide complex. Preferably, the MHC binding peptide is joined to the N-terminus of the beta chain. Essentially any MHC binding peptides may be joined to the N-termini of MHC Class II chains with which they selectively bind in nature. In particularly preferred embodiments with medical importance to multiple sclerosis, however, the MHC Class II molecule is an HLA-DR2 molecule and the binding peptide is selected from residues 85-99, 84-102 and 148-162 of human myelin basic protein. Similarly, in particularly preferred embodiments with medical importance to pemphigus vulgaris, the MHC Class II molecule is an HLA-DR4 or HLA-DQ1 molecule and said binding peptide is selected from residues 78-93, 97-111, 190-204, 206-220, 251-265, 512-526 and 762-786 of the human desmoglein 3 protein.

In each of the foregoing embodiments employing a covalently bound MHC binding peptide in the fusion protein, a flexible molecular linker optionally may be interposed between, and covalently join, the MHC Class II chain and the MHC binding peptide. Preferably, the linker is a polypeptide sequence of 10-20 amino acid residues, more preferably 12-18 amino acid residues. When a polypeptide linker is employed, it is preferred that a majority of the amino acid residues are alanine, glycine, serine, leucine, isoleucine, and valine residues.

In another aspect, the present invention provides Class II Major Histocompatibility Complex fusion proteins comprising a heterodimer of a first polypeptide chain and a second polypeptide chain; in which the first polypeptide chain comprises a fusion of, toward the N-terminus, at least an extracellular domain of an MHC Class II α chain and, toward the C-terminus, a first dimerization domain, and the second polypeptide chain comprises a fusion of, toward the N-terminus, at least an extracellular domain of an MHC Class IT β chain and, toward the C-terminus, a second dimerization domain. In these embodiments, the first dimerization domain and the second dimerization domain associate in solution at physiological conditions to form a heterodimer capable of selectively binding an MHC binding peptide. The dimerization domains, as described above, may be coiled-coil dimerization domains and, preferably, leucine zipper domains. Flexible molecular linkers, as described above, may be interposed between and covalently join the MHC chains and dimerization domains, and MHC binding peptides may be covalently joined to one of the MHC chains.

In another aspect, which does not form part of the claimed invention, a Class II Major Histocompatibility Complex fusion protein is provided comprising a heterodimer of a first polypeptide chain and a second polypeptide chain, in which the first polypeptide chain comprises a fusion of, toward the N-terminus, at least an extracellular domain of an MHC Class II α chain and, toward the C-terminus, an immunoglobulin heavy chain C_{H}1 constant region, and the second polypeptide chain comprises a fusion of, toward the N-terminus, at least an extracellular domain of an MHC Class II β chain and, toward the C-terminus, an immunoglobulin light chain constant region. In these embodiments, the immunoglobulin heavy chain C_{H}1 constant region and the immunoglobulin light chain constant region dimerize in solution at physiological conditions to form a heterodimer capable of selectively binding an MHC binding peptide. Alternatively, a Class II Major Histocompatibility Complex fusion protein is provided comprising a heterodimer of a first polypeptide chain and a second polypeptide chain, in which the first polypeptide chain comprises a fusion of, toward the N-terminus, at least an extracellular domain of an MHC Class II α chain and, toward the C-terminus, an immunoglobulin light chain constant region, and the second polypeptide chain comprises a fusion of, toward the N-terminus, at least an extracellular domain of an MHC Class II β chain and, toward the C-terminus, an immunoglobulin heavy chain C_{H}1 constant region. In these embodiments, as above, the immunoglobulin heavy chain C_{H}1 constant region and the immunoglobulin light chain constant region dimerize in solution at physiological conditions to form a heterodimer capable of selectively binding an MHC binding peptide. In each of these embodiments, the Class II Major Histocompatibility Complex fusion protein may further comprise an immunoglobulin Fc region covalently joined to the immunoglobulin heavy chain C_{H}1 constant region. Such Fc regions may be IgE or IgM Fc regions, and a flexible molecular linker may optionally be interposed between, and covalently join, the immunoglobulin heavy chain C_{H}1 constant region and immunoglobulin Fc region. Alternatively, the Fc regions may be IgA, IgD or IgG Fc regions. As before, a flexible molecular linker may be optionally interposed between, and covalently join, the immunoglobulin heavy chain C_{H}1 constant region and immunoglobulin Fc region and, in these embodiments, may be immunoglobulin hinge regions. In further embodiments which do not form part of the clamed invention a multivalent Class II Major Histocompatibility Complex fusion protein is provided comprising two Class II Major Histocompatibility Complex fusion proteins as described above, in which the Fc regions are covalently joined by at least one disulfide bond. Most preferably, a multivalent Class II Major Histocompatibility Complex fusion protein is provided comprising five pairs of Class II Major Histocompatibility Complex fusion proteins in which the Fc regions are IgM regions, each pair is covalently joined by at least one disulfide bond between Fc regions of each pair, and the five pairs are covalently joined by disulfide bridges to form a ring structure such that each adjacent pair in the ring is joined by at least one disulfide bond.

In each of the foregoing embodiments, the Class II Major Histocompatibility Complex fusion protein may further comprise an N-terminal secretory signal sequence covalently joined to the N-terminus of the fusion protein. In preferred embodiments. the secretory signal sequence comprises a yeast α-mating factor secretion signal or a human MHC Class II protein secretion signal.

In another aspect, the present invention provides a soluble monovalent or multivalent MHC/peptide complex of the present invention for use in a therapeutic method.

In another aspect, the present invention provides a method for detecting T cells having a defined Class II MHC-peptide complex specificity comprising providing a monovalent or multivalent Class II Major Histocompatibility Complex fusion protein, as described above and comprising the defined Class II MHC-peptide complex, contacting a population of T cells with the fusion protein, and detecting the presence or absence of binding of the fusion protein and T cells in the population. Also provided is a method further comprising isolating T cells reactive with the defined MHC-peptide complex from the population ofT cells by, for example, means of fluorescence activated cell sorting.

In another aspect, the present invention provides a soluble monovalent or multivalent MHC/peptide complex of the present invention for conferring to a subject adoptive immunity to a target protein comprising said MHC binding peptide.

In another aspect, the present invention provides an in vitro method for stimulating or activating T cells reactive to a defined Class II MHC-peptide complex comprising providing a monovalent or multivalent Class II Major Histocompatibility Complex fusion protein, as described above and comprising the defined Class II MHC-peptide-complex, and contacting a population of T cells with an immunogenic amount of the fusion protein.

In another aspect,the present invention provides a soluble monovalent or multivalent MHC/peptide complex of the present invention for stimulating or activating T cells reactive to a defined MHC/peptide complex.

In preferred embodiments, the fusion protein is contacted with the population of T cells in vivo in a human subject, and the Class II MHC fusion protein comprises a Class II MHC binding domain which is syngeneic to the subject.

In another aspect, which does not form part of the claimed invention, a method for selectively killing T cells reactive to a defined Class II MHC-peptide complex is provided comprising providing a monovalent or multivalent Class II Major Histocompatibility Complex fusion protein, as described above and comprising the defined Class II MHC-peptide-complex, and contacting a population of T cells with the fusion protein, in which the fusion protein comprises a domain of an immunoglobulin effective to activate a complement system and cause the complement system to kill the T cells.

In another aspect, the present invention provides a *invitro* method for selectively killing T cell reactive to a defined Class II MHC-peptide complex comprising providing a monovalent or multivalent Class II Major Histocompatibility Complex fusion protein, as described above and comprising the defined Class II MHC-peptide-complex, and contacting a population of T cells with the fusion protein, in which the fusion protein comprises a cytotoxic substance covalently bound to the protein and capable of killing T cells to which the fusion protein selectively binds.

In another aspect, the present invention provides a soluble monovalent or multivalent MHC/peptide complex of the present invention, for selectively killing T cells reactive to a defined MHC/peptide complex, wherein said MHC/peptide complex further comprises a covalently bound cytotoxic substance.

In another aspect, the present invention provides a soluble monovalent or multivalent MHC/peptide complex of the present invention, for tolerizing a human subject to a defined MHC Class II binding pepude. In certain preferred embodiments, the Class II MHC fusion protein comprises a Class II MHC binding domain which is syngeneic to the subject. In other preferred embodiments, however, the Class II MHC fusion protein comprises a Class II MHC binding domain which is allogeneic to the subject.

In another aspect the present invention provides nucleic acid sequences encoding the above-described Class II MHC Fusion proteins.

### Brief Description of the Drawings

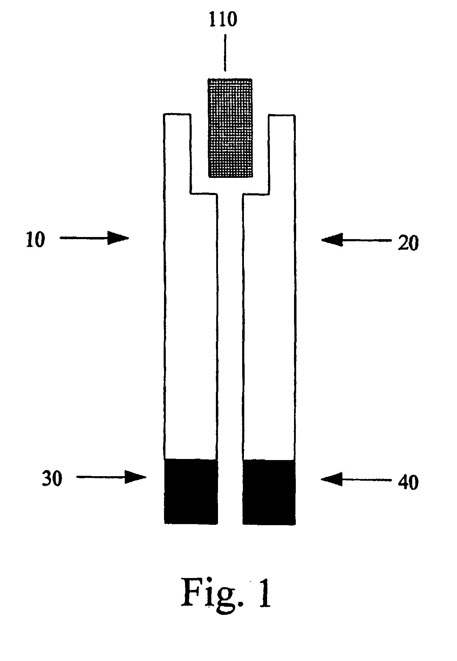

Figure 1. This figure is a schematic representation of a monovalent MHC fusion protein of the invention. Here, an extracellular or peptide binding domain of an MHC Class II α chain **10** has been joined to a first coiled-coil dimerization domain **30**, an extracellular or peptide binding domain of an MHC Class II β chain **20** has been joined to a second coiled-coil dimerization domain **40**, and these two fusion constructs have assembled to form a heterodimeric molecule which may bind an MHC binding peptide **110** in the cleft formed by the MHC Class II binding domains of the α and β chains, **10** and **20**. Flexible molecular linkers, not shown, optionally may be interposed between the MHC domains (**10**, **20**) and the dimerization domains **(30, 40).**

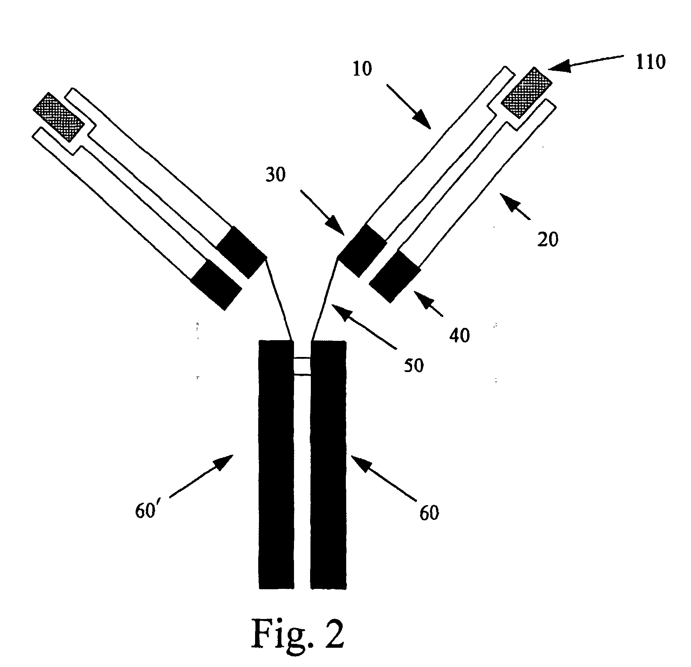

Figure 2. This figure is a schematic representation of a divalent MHC-immunoglobulin fusion protein construct of the invention. Here, an extracellular or peptide binding domain of an MHC Class II α chain 10 has been joined to a first coiled-coil dimerization domain **30**, and an extracellular or peptide binding domain of an MHC Class II β chain **20** has been joined to a second coiled-coil dimerization domain **40.** As shown, the domain **30** fused to the MHC α chain domain **10** is further fused to the hinge region **50** (optional) and Fc region **60** of an immunoglobulin chain Alternatively, not shown, the MHC α and β chain domains **10** and **20** may be switched such that the MHC β chain domain is fused to the immunoglobulin heavy chain domains **50** and **60.** The dimerization domains **30** and **40** promote the assembly of these two fusion constructs to form a heterodimeric structure which may bind an MHC binding peptide **110** in the cleft formed by the MHC Class II binding domains of the α and β chains, **10** and **20**. Flexible molecular linkers, not shown, optionally may be interposed between the MHC domains (**10, 20**) and the dimerization domains **(30, 40),** and/or between the dimerization domain **30** and the immunoglobulin hinge **50** or Fc region **60.** The Fc regions **60** and **60'** of two of these heterodimeric MHC-immunoglobulin fusion proteins have associated in the manner of an antibody to form a divalent MHC fusion protein construct. Horizontal lines between the Fc regions **60** and **60'** represent disulfide bridges between the immunoglobulin heavy,chain domains.

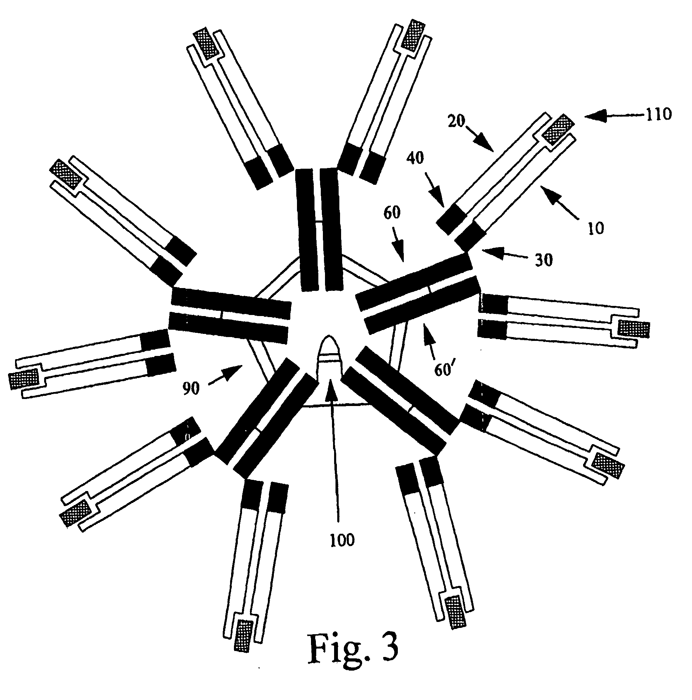

Figure 3. This figure is a schematic representation of a decavalent MHC-IgM immunoglobulin fusion protein construct of the invention. Here, an extracellular or peptide binding domain of an MHC Class II α chain **10** has been joined to a first coiled-coil dimerization domain **30**, an extracellular or peptide binding domain of an MHC Class II β chain **20** has been joined to a second coiled-coil dimerization domain **40**, and these two fusion constructs have assembled to form a heterodimeric molecule which may bind an MHC binding peptide **110** in the cleft formed by the MHC Class II binding domains of the α and β chains, **10** and **20**. As shown, the domain **30** fused to the MHC α chain domain **10** is further fused to an IgM Fc domain (C_{H}2, C_{H}3, C_{H}4) **60**. Alternatively, not shown, the MHC α and β chain domains **10** and **20** may be switched such that the MHC β chain domains are fused to the immunoglobulin heavy chain domains **60**. The Fc regions **60** and **60'** of two heterodimeric MHC-immunoglobulin fusion proteins have associated in the manner of a single IgM subunit to form a divalent MHC-IgM fusion structure joined by a disulfide bond. Five of these divalent MHC-IgM fusion subunits have assembled to form a characteristic IgM pentamer, joined by disulfide bonds **90** between IgM subunits and including a J-chain peptide 100, and resulting in a decavalent MHC-IgM fusion structure. Flexible molecular linkers, not shown, optionally may be interposed between the MHC domains **(10, 20)** and the coiled-coil domains **(30, 40),** and/or between the domains **(30)** and the IgM Fc domains **(60).**

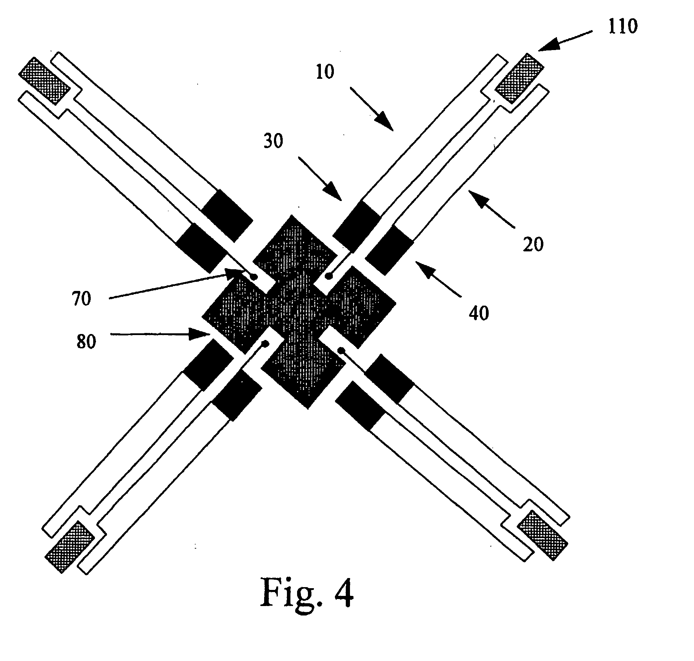

Figure 4. This figure is a schematic representation of a tetravalent MHC-tag fusion protein construct of the invention. Here, an extracellular or peptide binding domain of an MHC Class II α chain **10** has been joined to a first coiled-coil dimerization domain **30**, an extracellular or peptide binding domain of an MHC Class II β chain **20** has been joined to a second coiled-coil dimerization domain **40,** and these two fusion constructs have assembled to form a heterodimeric molecule which may bind an MHC binding peptide **110** in the cleft formed by the MHC Class II binding domains of the α and β chains, **10** and **20**. As shown, the domain **30** fused to the MHC α chain domain **10** is further fused to a biotinylated sequence tag **70** which binds to avidin or streptavidin **80.** Alternatively, not shown, the MHC α and β chain domains **10** and **20** may be switched such that the MHC β chain domain is fused to the biotinylated sequence tag **70**. Because each avidin or streptavidin molecule may bind four biotin moieties, these MHC-tag-biotin/(strept)avidin complexes are tetravalent. Flexible molecular linkers, not shown, optionally may be interposed between the MHC domains (**10, 20**) and the dimerization domains **(30, 40),** and/or between the dimerization domain **30** and the biotinylated sequence tag **70**.

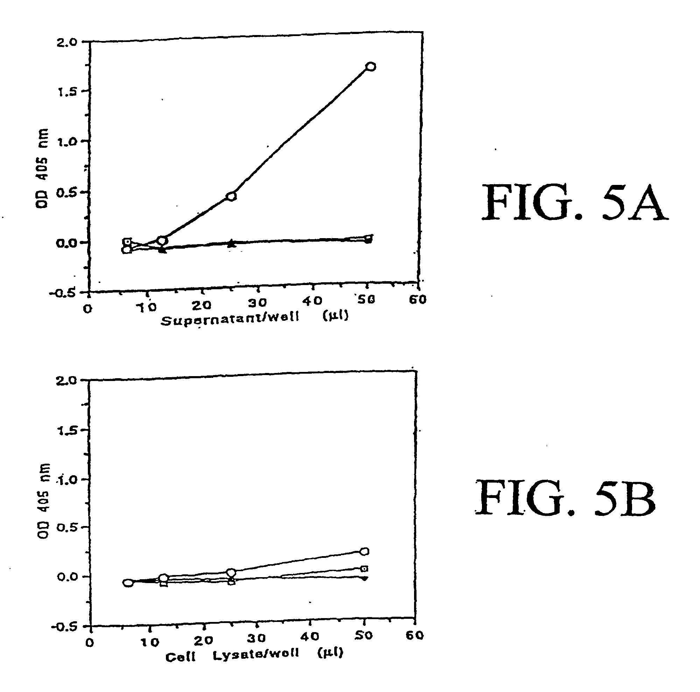

Figure 5. This figure graphically presents the results of experiments demonstrating the assembly and secretion of recombinant HLA-DR2 fusion proteins by Pichia pastoris. Expression of DR2 fusion proteins (DRα-Fos and DRβ-Jun) were examined by sandwich ELISA of cell culture supernatants (top graph) or cell culture lysates (bottom graph) using a mAb (L243) specific for the DR2 αβ heterodimer for capture, and a polyclonal DR antiserum for detection. Binding of the secondary antibody was quantitated with a peroxidase conjugated anti-rabbit IgG antiserum, with ABTS as the peroxidase substrate and detection at 405 nm. Results are from cells transfected with: DRα-Fos only, open squares; DRβ-Jun only, solid circles; and both DRα-Fos and DRβ-Jun, open circles.

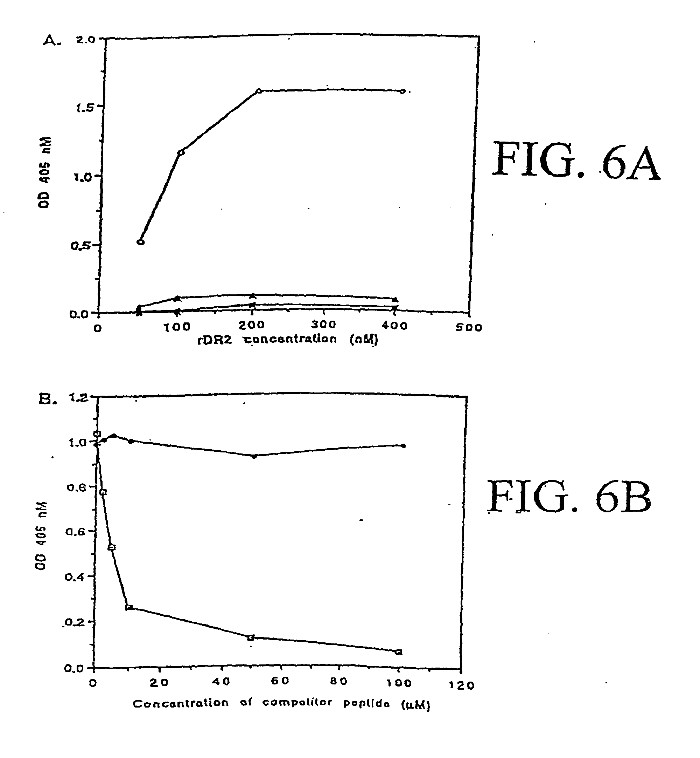

Figure 6. This figure presents the results of experiments demonstrating the specificity of peptide binding to recombinant HLA-DR2 (rDR2) fusion proteins. Peptide binding was examined using a biotinylated MBP(85-99) peptide ("MBP") that was previously shown to bind with high affinity to detergent soluble DR2. rDR2-MBP complexes were captured on an ELISA plate using a DR specific mAb (L243) and DR bound biotinylated MBP was quantitated using peroxidase labeled streptavidin, with ABTS as the peroxidase substrate and detection at 405 nm. The top graph shows the effect of rDR2 concentration on peptide binding with: 2 µM biotinylated MBP peptide, open circles; 2µM biotinylated MBP peptide with 100 µM unbiotinylated MBP as a competitor, solid triangles; and no peptide, solid squares. The same ELISA assay was used with 200 nM rDR2 and 2 µM biotinylated MBP to demonstrate binding specificity. The bottom graph shows the effect of varying concentrations of competitor peptides on biotinylated MBP peptide binding to rDR2 fusion proteins: unbiotinylated MBP competitor, open squares; Val89→Asp MBP competitor, closed circles.

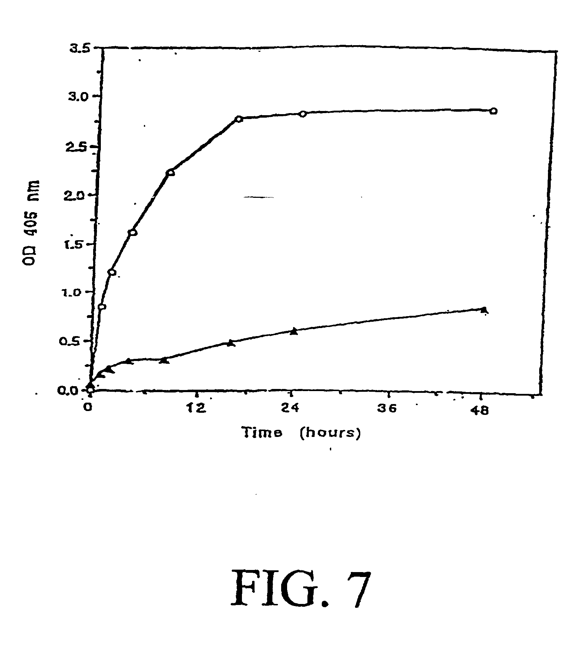

Figure 7. This figure presents the results of experiments demonstrating the kinetics of peptide binding to recombinant HLA-DR2 fusion proteins (rDR2). The kinetics of peptide binding were compared for rDR2 and for detergent soluble DR2 purified from an EBV transformed B cell line. The DR2 proteins (200 nM) were incubated with biotinylated MBP peptide (2 µM) at 37°C for different periods of time; the amount of DR bound peptide was examined by ELISA using a DR specific antibody for capture and streptavidin-peroxidase for quantification of bound peptide, with ABTS as the peroxidase substrate and detection at 405 nm. The graph shows biotinylated MBP peptide binding over time for: recombinant DR2 fusions, open squares; detergent solubilized B cell DR2 molecules, closed triangles.

### Detailed Description of the Invention

### I. Definitions.

In order to more clearly and concisely describe and point out the subject matter of the claimed invention, the following definitions are provided for specific terms which are used in the following description and the claims appended hereto.

As used herein, the term "MHC Class II" or "Class II" refers to the human Major Histocompatibility Complex Class II proteins, binding peptides or genes. The human MHC region, also referred to as HLA, is found on chromosome six and includes the Class I region and the Class II region. Within the MHC Class II region are found the DP, DQ and DR subregions for Class II α chain and β chain genes (i.e., DPα, DPβ, DQα, DQβ, DRα, and DRβ). As used herein, the term "MHC Class II molecule" means a covalently or non-covalently joined complex of an MHC Class II α chain and an MHC Class II β chain. MHC class II molecules bind peptides in an intracellular processing compartment and present these peptides on the surface of antigen presenting cells to T cells. Peptides are bound in an extended conformation, as left-handed type II polyproline helices. The majority of bound peptides have a length of 13-18 amino acids but it is the peptide side chains of an approximately nine amino acid core segment (P1 to P9) that occupy pockets of the MHC Class II binding domain and determine the specificity of binding (Brown et al., 1993; Stem et al., 1994).

As used herein, the term "MHC Class II α chain" means a naturally occurring polypeptide, or one encoded by an artificially mutated α gene, essentially corresponding to at least the α₁ and α₂ extracellular domains of one of the gene products of an MHC Class II α gene (e.g., a DP, DQ or DR α gene). As the C-terminal transmembrane and cytoplasmic portions of the α chain are not necessary for antigenic peptide binding in the present invention, they may be omitted while retaining biological activity. In addition, the term "MHC Class II α chain" is intended to include variants with and without the usual glycosylation of the α₁ and α₂ domains. The term is particularly intended to embrace all allelic variants of the Class II α genes, as well as any equivalents which may be produced synthetically or recombinantly by, for example, site-directed mutagenesis of a naturally occurring variant.

As used herein, the term "MHC Class II β chain" means a naturally occurring polypeptide, or one encoded by an artificially mutated β gene, essentially corresponding to at least the β₁ and β₂ extracellular domain of one of the gene products of an MHC Class II β gene (e.g., DP, DQ or DR β gene). As the C- terminal transmembrane and cytoplasmic portions of the β chain are not necessary for antigenic peptide binding in the present invention, they may be omitted while retaining biological activity. In addition, the term "MHC Class II β chain" is intended to include variants with and without the usual glycosylation of the β₁ domain. The term is particularly intended to embrace all allelic variants of the Class II β genes, as well as any equivalents which may be produced synthetically or recombinantly by, for example, site-directed mutagenesis of a naturally occurring variant.

As used herein, the term "MHC Class II binding domain" refers to the regions of the MHC Class II molecules which are necessary for binding an antigenic peptide. The MHC Class II binding domain is formed primarily by the α₁ and β₁ domains of the MHC Class II α and β chains and, therefore, an MHC Class II binding domain minimally includes these regions. The α₂ and β₂ domains of these proteins, however, are also believed to be important to stabilizing the over-all structure of the MHC binding cleft and, therefore, an MHC Class II binding domain of the present invention preferably includes these regions. An MHC Class II binding domain may also be essentially defined as the extracellular domain of an MHC Class II molecule, distinguishing it from the transmembrane and cytoplasmic domains, although it is likely that some portion of the extracellular domain may be omitted while retaining biological activity.

As used herein, the term "MHC Class II binding peptide" means a polypeptide which is capable of selectively binding within the cleft formed by the α and β chains of a specified MHC Class II molecule to form an MHC Class II-peptide antigen complex. An MHC Class II binding peptide may be a processed self or non-self peptide or may be a synthetic peptide. For MHC Class II proteins, the peptides are typically 10-25 amino acids in length, and more typically 13-18 residues in length, although longer and shorter ones may be effective.

As used herein, the term "MHC/peptide complex" means a covalently or non-covalently joined ternary complex of the binding domain of an MHC Class II molecule and an MHC Class II binding peptide which binds to that MHC Class II binding domain.

As used herein, the term "flexible molecular linker" or "linker" means a chemical moiety having a length equal to or greater than that of three carbon to carbon bonds and including at least one freely rotating bond along said length. Preferably, a flexible molecular linker is comprised of one or more amino acid residues but this need not be the case. In certain preferred embodiments, the flexible molecular linkers of the invention comprise at least three and, more preferably, at least seven amino acid residues.

As used herein, the term "selectively binding" means capable of binding in the electro- and stereospecific manner of an antibody to antigen or ligand to receptor. With respect to an MHC binding peptide, selective binding entails the non-covalent binding of specific side chains of the peptide within the binding pockets present in the MHC binding domain in order to form an MHC-peptide complex (see, e.g., Brown et al., 1993; Stern et al., 1994).

As used herein, the term "substantially pure" means, with respect to the MHC binding peptides and various fusion proteins of the invention, that these peptides or proteins are essentially free of other substances to an extent practical and appropriate for their intended use. In particular, the peptides and proteins are sufficiently pure and are sufficiently free from other biological constituents of their hosts cells so as to be useful in, for example, generating antibodies or producing pharmaceutical preparations. A substantially pure preparation of the peptides or proteins of the invention need not be absolutely free of all other proteins or cell components and, for purposes of administration, may be relatively dilute. One of ordinary skill in the art may produce such substantially pure preparations by application or serial application of well-known methods including, but not limited to, HPLC, affinity chromatography or electrophoretic separation. The substantially pure preparations of the invention may also comprise other active ingredients and, therefore. the percentage by weight of the MHC binding peptides and/or various MHC fusion proteins of the invention may be reduced in such a preparation.

### II. Preferred Embodiments

The present invention depends, in part, upon the discovery that fusion proteins, comprising MHC Class II protein domains and coiled-coil , may be recombinantly produced, and that these fusion proteins may form heterodimers including biologically functional MHC Class II binding domains in monovalent or multivalent proteins. In particular, it is disclosed that (1) extracellular domains of certain MHC Class II molecules which previously could not be produced as empty, soluble, stable heterodimers, may be produced using fusion proteins incorporating coiled-coil dimerization domains, and (2) heterodimeric MHC Class II binding domains may be produced in the form of multivalent proteins by incorporating them as fusions in multivalent immunoglobulin or biotin/streptavidin structures. Of particular importance, is the surprising result that the MHC Class II binding domains of these fusion proteins retain their biological activity despite the functional requirement for highly specific tertiary and quaternary structural interactions within and between the α and β chains of the MHC molecule, and despite the substitution of relatively large, relatively hydrophilic fusion domains for the natural, hydrophobic transmembrane domains of the MHC Class II proteins.

Thus, in a first series of embodiments, the present invention provides for the production of fusion proteins of MHC Class II α and β chain proteins in which substantially all of the C-terminal transmembrane and cytoplasmic domains have been replaced by coiled-coil dimerization domains and, optionally, interposing linker sequences. Figure 1 schematically illustrates such a monovalent MHC Class II fusion protein. At least the peptide binding domain, and preferably the entire extracellular domain, of an MHC Class II α chain **10** may be fused to a first dimerization domain **30** (e.g., a leucine zipper domain). Similarly, at least the peptide binding domain, and preferably the entire extracellular domain, of an MHC Class II β chain **20** may be fused to a second dimerization domain **40** (e.g., a leucine zipper domain)

The dimerization domains (**30** and **40**) associate in solution to promote formation of a heterodimeric fusion protein in which the MHC Class II α and β chain components (**10** and **20**) stably associate to form a biologically active MHC Class II binding domain which is capable of binding, or being "loaded," with MHC binding peptides **110** so as to form a stable MHC/peptide complex which can selectively bind to the cognate T cell receptors and selectively activate T cell clones bearing the cognate TCRs Optionally, flexible molecular linkers may be interposed between the MHC components (**10** and **20**) and dimerization domains (**30** and **40**) so as to better approximate the normal distance between the MHC components and their natural MHC transmembrane domains, and/or to provide for free rotation between the MHC components and the dimerization domains such that the geometry of the association between dimerization domains does not constrain or interfere with the geometry of association of the MHC domains.

In another series of embodiments, the present invention provides for the production of divalent fusion proteins of MHC Class II α and β chain proteins in which substantially all of the C-terminal transmembrane and cytoplasmic domains have been replaced by coiled-coil dimerization domains and optionally, interposing linker sequences.

The immunoglobulin constant domains are chosen so as to promote the formation of divalent antibody-like molecules bearing two MHC Class II binding domains and, optionally, to promote certain effector functions (e.g., complement activation, cell binding). Figure 2 schematically illustrates such a divalent MHC Class II fusion protein. At least the peptide binding domain, and preferably the entire extracellular domain, of an MHC Class II α chain 10 may be fused to a first dimerization domain 30 (e.g., a leucine zipper domain)

Similarly, at least the peptide binding domain, and preferably the entire extracellular domain, of an MHC Class II β chain **20** may be fused to a second dimerization domain **40** (e.g., a leucine zipper domain)

The dimerization domains (**30** and **40**) associate in solution to promote formation of a heterodimeric fusion protein in which the MHC Class II α and β components (**10** and **20**) stably associate to form a biologically active MHC Class II binding domain which is capable of binding, or being "loaded," with MHC binding peptides **110** so as to form a stable MHC/peptide complex which can selectively bind to the cognate T cell receptors and selectively activate T cell clones bearing the cognate TCRs. As noted above, however, some MHC Class II molecules (e.g., HLA-DR1, HLA-DR4) can be expressed by the method of Stern and Wiley (1992) as stable, soluble heterodimers without their transmembrane and cytoplasmic domains. Next, one of the two MHC fusion proteins further comprises an immunoglobulin Fc region **60**, with or without an interposing immunoglobulin hinge region **50** appropriate to the Fc region (IgA, IgD and IgG molecules include hinge regions; IgE and IgM molecules do not). Preferably, it is the MHC Class II α chain fusion protein which is fused to the immunoglobulin heavy chain Fc region because the MHC Class II α chains are less variable than the β chains and, therefore, such an α chain fusion protein could be used with a number of different MHC Class II β chain fusion proteins to form a variety of different divalent molecules with different HLA specificity. It should, however, be noted that there is no reason that the β chain construct could not include the immunoglobulin Fc regions. Finally, flexible molecular linkers may be optionally interposed between the MHC components (**10** and **20**), dimerization domains (**30** and **40**), and/or immunoglobulin components (**50** and/or **60**) so as to better approximate the normal distance between the MHC components and their natural MHC transmembrane domains, and/or to provide for free rotation between the MHC components, the dimerization domains, and/or the immunoglobulin domains such that the geometry of the association between any pair of dimerizing components does not constrain or interfere with the geometry of association or dimerization of the others. As shown in Figure 2, The immunoglobulin heavy chain Fc regions 60 and 60' of two such MHC Class II fusion proteins associate and form a divalent structure, with one or more disulfide linkages between chains, analogous to the structure of natural antibodies.

In another series of embodiments, the present invention provides for the production of decavalent fusion proteins of MHC Class II α and β chain proteins in which substantially all of the C-terminal transmembrane and cytoplasmic domains have been replaced by coiled-coil dimerization domains and, optionally, interposing linker sequences.

These embodiments are essentially the same as those described immediately above except that (1) the immunoglobulin constant domains are specifically chosen to be IgM chains, which form divalent subunits which are then assembled into decavalent pentamers, and (2) that the cells producing these MHC-IgM fusions will be cotransfected with a J-chain gene in order to facilitate the assembly and secretion of IgM molecules (Matsuuchi et al., 1986). Figure 3 schematically illustrates such a decavalent MHC Class II fusion protein. As before, at least the peptide binding domains, and preferably the entire extracellular domains, of MHC Class II α **10** and β **20** chains may be fused to dimerization domains **30** and **40** (e.g., a leucine zipper domain). The dimerization domains (**30** and **40**) associate in solution to promote formation of a heterodimeric fusion protein in which the MHC Class II α and β components (**10** and **20**) stably associate to form a biologically active MHC Class II binding domain which is capable of binding, or being "loaded," with MHC binding peptides **110**. Next, as above, either the α or β chain construct further comprises an immunoglobulin Fc region 60 which, in these embodiments, is an IgM Fc region (C_{H}2, C_{H}3, C_{H}4). Finally, as before, flexible molecular linkers may be optionally interposed between the MHC components (**10** and **20**), dimerization domains (**30** and **40**), and/or IgM Fc components (**60**) so as to better approximate the normal distance between the MHC components and their natural MHC transmembrane domains, and/or to provide for free rotation between the MHC components, the dimerization domains, and/or the immunoglobulin domains. As shown in Figure 3, the immunoglobulin heavy chain Fc regions **60** and **60'** of two such MHC-IgM fusion proteins are associated to form a divalent structure with one or more disulfide linkages between chains. These divalent subunits, however, will further associate to form a multimer with one or more disulfide bonds **90** between divalent subunits. In the presence of the J-chain protein **100**, IgM subunits are assembled into decavalent pentamers as shown in Figure 3, analogous to naturally occurring IgM pentamers.

Finally, in another series of embodiments, the present invention provides for the production of tetravalent fusion proteins ofMHC Class II α and β chain proteins in which substantially all of the C-terminal transmembrane and cytoplasmic domains have been replaced by coiled-coil dimerization domains and, optionally, interposing linker sequences, and in which a biotinylated C-terminal "tag" sequence allows up to four MHC-tag fusions to bind to avidin (or streptavidin) and form a tetravalent MHC fusion protein complex. Figure 4 schematically illustrates such a tetravalent MHC fusion protein complex. As in previous embodiments, at least the peptide binding domain, and preferably the entire extracellular domain, of MHC Class II α chains **10** and β chains **20** may be fused to dimerization domains (**30** and **40**) (e.g., a leucine zipper domain). The dimerization domains (**30** and **40**) associate in solution to promote formation of a heterodimeric fusion protein in which the MHC Class II α and β components (**10** and **20**) stably associate to form a biologically active MHC Class II binding domain which is capable of binding, or being "loaded," with MHC binding peptides **110**. In addition, a biotin ligase recognition sequence or "tag" is fused to the C-terminus of at least one of the MHC-fusion chains. This sequence tag may be biotinylated by enzymes within the cells which produce these MHC fusion proteins, or may be subsequently biotinylated in vitro. The biotinylated tag **70** can be used to cause the monovalent MHC fusion proteins to bind to avidin (or streptavidin) **80.** As each avidin (or streptavidin) molecule is capable of binding up to four biotin moieties, an MHC-biotin/(strept)avidin fusion protein complex can be produced which is tetravalent (with lower valences at lower molar ratios of biotin:(strept)avidin). As before, flexible molecular linkers may optionally be interposed between the MHC components (**10** and **20**), the dimerization domains (**30** and **40**) and/or the biotin sequence tag so as to better approximate the normal distance between the MHC components and their natural MHC transmembrane domains, and/or to provide for free rotation between the MHC components, the dimerization domains, and/or biotinylated tag. As will be apparent to one of ordinary skill in the art, other sequence tags and ligand complexes may be employed instead ofbiotin/(strept)avidin to produce similar multimeric MHC fusion protein complexes.

In each of the foregoing embodiments, the M<HC binding peptide 110 may be covalently joined to either the MHC Class II α or β components (**10** and **20**) with a flexible molecular linker (not shown in the Figures). Preferably, such flexible molecular linkers are polypeptide sequences of 10-20 amino acid residues, more preferably 12-18 amino acid residues. When the flexible molecular linkers are polypeptides, the MHC binding peptide, linker and MHC Class II α or β components may all be expressed as a single fusion protein, further comprising dimerization domains toward the C-terminus.

In connection with each of the above-described embodiments, the present invention provides (a) isolated nucleic acid sequences encoding such fusion proteins; (b) vectors for transiently or stably transfecting host cells with these nucleic acids; (c) host cells transformed with these sequences or vectors; (d) methods for producing the fusion proteins employing these sequences, vectors and host cells; and (e) the substantially purified fusion proteins themselves. In addition, the present invention provides for a number of utilities of these products and processes including, but not limited to, the treatment of allergic and autoimmune diseases, the detection and/or isolation of T cells with defined MHC-peptide specificities, and the selective activation, anergization, or killing of T cells with defined MHC-peptide specificities.

### A. Choice of MHC Components

The methods and products of the present invention may be practiced with any mammalian MHC Class II proteins. Primarily, however, it is anticipated that the present invention will have greatest utility in the diagnosis and treatment of human disease and, therefore, the MHC Class II proteins are preferably human HLA Class II proteins. Thus, for example, the present invention may be practiced with either of the known HLA-DRA alleles (DRA*0101 and DRA*0102), any of the approximately 160 known HLA-DRB alleles (including at least 137 known HLA-DRB1 alleles), any of the approximately 15 known HLA-DQA1 alleles, any of the approximately 25 known HLA-DQB1 alleles, any of the approximately 8 known HLA-DPA1 alleles, or any of the approximately 65 known HLA-DPB1 alleles. A compilation of known human HLA Class II nucleotide sequences has been published by Marsh and Bodmer (1995), and a compilation of known HLA Class II nucleotide and amino acid sequences is available via electronic transfer from the EMBL Data Library, Cambridge, UK (request "HELP HLA" by e-mail to "netserv@ebi.ac.uk"). All of these sequences are not, therefore, reproduced herein. In addition, all sequence nomenclature used herein conforms to that used in Marsh and Bodmer (1995), and in Bodmer et al. (1995).

Embodiments employing coiled-coil dimerization domains are particularly preferred for use with those MHC Class II binding domains which, without their transmembrane and cytoplasmic domains, do not form stable heterodimers in solution. For these proteins, the coiled-coil domains add stability to the heterodimer while allowing for the production of soluble, non-aggregated proteins. Amongst these are the HLA-DR2 serotypes (e.g., those encoded by DRA and DRB1*15 or DRB1*16 alleles), HLA-DQ8 (encoded by, for example, the DQA1*0301 and DQB1*0302 alleles), and HLA-DQ2 (encoded by, for example, DQA1*0501 and DQB1*0201 alleles). Nonetheless, coiled-coil dimerization domains may be employed with any of the human MHC Class II binding domains, including those which have previously been successfully expressed as stable, soluble heterodimers without their transmembrane domains (e.g., DR1 and DR4).

### B. Choice of MHC Splice Points

In accordance with the present invention, splice points for the MHC components of the MHC Class II fusion proteins must be chosen so as to include sufficient N-terminal sequence for proper MHC binding domain formation while excluding most if not all of the C-terminal transmembrane and cytoplasmic domains of the MHC chains. As is well known in the art, the MHC Class II α and β chains are each characterized by two N-terminal, globular, extracellular domains (α1 and α2, or β1 and β2), followed by a short loop or connecting peptide, a hydrophobic transmembrane domain, and a C-terminal hydrophilic cytoplasmic domain. The binding cleft of the MHC Class II molecules is formed primarily by the interaction of the α1 and β1 domains in the heterodimer and, therefore, these domains must minimally be included in the fusion proteins of the present invention. The α2 and β2 domains, however, are also preferably included because they may aid in stabilizing the MHC binding domain, are believed to be involved in the formation of dimers of the MHC chains, and are believed to be involved in CD4 receptor binding.

Thus, in preferred embodiments, the splice points for the MHC Class II fusion peptides are chosen to be in the regions approximately between the ends of the α2 or β2 domains and the beginnings of the transmembrane domains. For most MHC Class II α chains, this corresponds to approximately amino acid residue positions 180-200 and for most β chains this corresponds to approximately amino acid residue positions 185-205. For example, for the HLA-DR α chains encoded by the DRA alleles (DRA*0101 or DRA*0102) the transmembrane domains essentially begin after the Glu residue at position 191 or the Asn residue at position 192. For the HLA-DR β chains encoded by the DR1 subtype DRB1*01 (e.g., DRB1*0101) alleles and the DR2 subtype DRB 1*15 and DRB 1*16 alleles (e.g., DRB 1*1501), the transmembrane domains essentially begin after the Lys residue at position 198 or the Met residue at position 199. Similarly, for the DQ2 and DQ8 subtypes, the HLA-DQA1 transmembrane domains essentially begin after the Glu residue at position 195 or the Thr residue at position 196, and the HLA-DQB1 transmembrane domains essentially begin after the Lys residue at position 200 or the Met residue at position 201. For some allelic variants, of course, there may be amino acid insertions or deletions prior to these sites which alter the residue numbering. The connecting peptide and transmembrane regions of the MHC Class II α and β chains are not, however, highly polymorphic and, indeed, appear invariant for all known DRA and DRB alleles (see Marsh and Bodmer, 1995). Therefore, working with any given MHC Class II α and β chains, one of ordinary skill in the art can easily identify the transmembrane domains both by homology to the above-described alleles, and by their essential hydrophobic nature.

Although not preferred, it is acceptable that the fusion proteins of the present invention include several residues from the transmembrane domain or that several residues of the α2 or β2 domains be omitted. For example, the inclusion of 1-5 residues of the transmembrane domain may be included in the present invention and still yield a soluble fusion protein but this is not preferred. Similarly, the omission of, for example, 1-5 residues from the α2 or β2 domains may not result in structural alterations which disrupt MHC peptide binding, heterodimer formation, or T cell interactions. Indeed, if replaced by suitable residues which conserve the over-all structure of the MHC molecule, larger portions of the α2 and β2 structural domains may be omitted in accordance with the present invention (e.g., replacing portions of the Class II α2 or β2 domains with equivalent portions of the Class I α3 or β2-microglobulin proteins). Thus, recognizing that one may also include or omit all or part of the 5-7 amino acid loop or connecting peptide which naturally joins the α2 and β2 domains to their respective transmembrane domains, one may produce fusion proteins in which the splice point is anywhere from approximately residues 180-200 of the α chain and approximately residues 185-205 of the β chain, with larger C-terminal omissions tolerated if appropriate replacement sequences are provided. Finally, 1-5 residues may be omitted or substituted at the N-terminus of an MHC Class II α or β chain binding domain, although this is not recommended.

In preferred embodiments, however, the splice points are chosen to be within the loop or connecting peptide sequence near the N-terminal end of the transmembrane domain. In the most preferred embodiments, the entire extracellular domains of the MHC Class II α and β chains are included in the MHC fusion proteins of the invention.

### C. MHC Binding Peptide Fusions

In connection with any of the foregoing embodiments, one may also create a fusion protein in which an MHC binding peptide is covalently joined to the N-terminus of either the α or β chain such that the binding peptide is capable of selectively binding within the binding domain formed by the given MHC Class II α and β chains. Preferably, the MHC binding peptide is joined to the N-terminus of the beta chain because, when the alpha and beta chains associate to form a heterodimeric MHC molecule, the N-terminus of the beta chain is more accessible than the N-terminus of the alpha chain. In addition, the beta chains of MHC Class II molecules are more polymorphic than the alpha chains and, therefore, the specificity of an MHC binding domain is more dependent upon the beta chain which is included in the molecule.

The MHC binding peptide is preferably linked to the MHC binding domain using a flexible molecular linker, as described below. In preferred embodiments, the flexible molecular linker is a polypeptide sequence of approximately 10-20 amino acid residues, more preferably 12-18 amino acid residues, which joins the binding peptide and MHC binding domains by standard polypeptide linkages to form a larger fusion protein which may be encoded by a single nucleic acid construct and expressed as a single fusion protein. In addition, it is preferred that the amino acids be chosen from the relatively small residues (e.g., alanine, glycine, serine, leucine, isoleucine, valine) in order to minimize the potential for steric hindrance.

As noted above, the MHC binding peptide is chosen such that it is capable of selectively binding to the MHC molecule to which it is attached. Thousands of combinations of MHC binding peptides and MHC molecules are known in the art and can be identified by standard methods (see, e.g., Chicz et al., 1993). Of particular interest are those pairs of MHC binding peptides and MHC molecules which are implicated in diseases, including infections and autoimmune diseases. For example, specific MHC binding peptides derived from the human myelin basic protein (e.g., residues 85-99, 84-102 and 148-162) and particular MHC alleles (e.g., HLA-DR2 or DRA/DRB1*1501) have been implicated in the development of multiple sclerosis. Therefore, in one preferred embodiment, monovalent or multivalent MHC Class II fusion proteins are produced in which an immunogenic myelin basic protein (MBP) peptide is covalently joined by a polypeptide linker sequence to the N-terminus of the peptide binding domain of an HLA-DRB1* 1501 protein, and this fusion is covalently joined, with or without an interposing flexible molecular linker, to a dimerization domain. Such a fusion protein may then be dimerized with a corresponding HLA-DRA alpha chain fusion protein such that the MBP peptide binds in the cleft formed by association of the MHC Class II alpha and beta chains. Similarly, certain residues of the human desmoglein 3 protein (e.g., residues 78-93, 97-111, 190-204, 206-220, 251-265, 512-526 and 762-786) and certain MHC alleles (e.g., HLA-DR4 or DRA/DRB 1 *0402, and HLA-DQ 1 or DQA/DQB1*05032) have been implicated in the development of pemphigus vulgaris (see, e.g., WO 96/27387). For other autoimmune diseases, including rheumatoid arthritis (RA) and systemic lupus erythematosus (SLE), immunodominant self peptides may be identified which selectively bind to particular MHC Class II molecules. For each of these, monovalent or multivalent MHC Class II fusion proteins may be produced, having the autoimmunogenic MHC binding peptides covalently joined to the MHC binding domains, and these may be used, as further described below, in tolerizing or anergizing the immune response to the autoantigens.

### D. Choice of Linker Domains

In accordance with the present invention, MHC fusion proteins may be produced which optionally include flexible molecular linkers which covalently join, as described above, (1) MHC binding domains to dimerization domains; (2) dimerization domains to immunoglobulin Fc domains or biotin ligase "tag" domains; or (3) MHC binding peptides to MHC Class II binding domains. Appropriate linkers include, but are not limited to, short polypeptide chains which can be encoded with the MHC domains in recombinant DNA constructs. More generally, however, appropriate linkers include any relatively small (e.g., < 2 kDa, preferably < 1 kDa) organic chemical moieties which are flexible because they include at least one single bond located between the termini and about which there is free rotation. Thus, for example, bifunctional molecules (e.g., an α,ω-dicarboxylic acid or an α,ω-diamine) of a lower alkyl chain may be employed, and such flexible molecular linkers may be reacted with the C-termini of the MHC components and the N-termini of the coiled-coil, immunoglobulin or biotin components (or with reactive groups of the amino acid side chains of any of these). Many other cross-linking agents, of course, are well known in the art and may be employed as substantial equivalents.

Preferably, however, the flexible molecular linkers of the present invention comprise a series of amino acid residues which can be encoded in a fusion gene construct. For example, a linker of 1-15 generally small (e.g., alanine, glycine, serine, leucine, isoleucine, valine) amino acid residues, optionally including one or more hydrophilic residues (e.g., aspartate, glutamate, lysine), may be employed as a linker. The length of the linker may be chosen so as to maintain, approximately, the spacing naturally found between the MHC binding domains and transmembrane domains of the MHC proteins and, therefore, the length of the linker may depend upon whether some or all of the naturally occurring loop or connecting residues between the binding domains and transmembrane domains have been included or omitted. In addition, as will be apparent to one of skill in the art, linker sequences may be particularly chosen so as to introduce specific proteinase cleavage sites in the protein or, for ease of recombinant DNA manipulations, to introduce specific restriction endonuclease sites into the recombinant construct. Thus, for example, one may include the naturally occurring 5-7 amino acid loop or connecting peptides of an MHC molecule and also include a 5-7 amino acid linker. Alternatively, the included portion of the loop or connecting peptide may be varied, the linker length may be varied, or the loop peptide and/or linker may be omitted entirely. Using standard techniques of site-directed mutagenesis, or restriction and ligation of recombinant constructs with a variety of different endonucleases, one of ordinary skill in the art can easily produce many variations on the fusion protein constructs and, as described below, rapidly test them for TCR binding and/or T cell activation. Thus, although some presently preferred embodiments employ the entire extracellular domains of the MHC molecules joined by particular linkers to coiled-coil, immunoglobulin or biotin domains, the invention is not limited to such embodiments.

### E. Choice of Heterodimer-Forming Domains

Coiled-coils are common structural features of dimeric proteins in which two α-helical polypeptides ("coils") are twisted ("coiled") about each other to form a larger quaternary structure or "coiled-coil" (see, e.g., Hu et al., 1990; Oas and Endow, 1994). Indeed, the transmembrane regions of HLA-DR α and β chains are thought to be a helices that assemble as a coiled-coil within the hydrophobic environment of the cell membrane (Cosson and Bonifacino, 1992). Other coiled-coils, however, are hydrophilic and may be found in secreted, cytosolic and nuclear proteins. For example, "leucine zippers" are coiled-coil domains which are present in a large number of DNA binding proteins and which may mediate either homodimer or heterodimer formation (see, e.g., Ferré-D'Amaré et al., 1993; O'Shea et al, 1989; O'Shea et al., 1991). In addition, several researchers have now designed artificial coiled-coil domains, including pairs of basic and acidic amphipathic helices and artificial leucine zippers, which have been expressed and assembled in recombinant homodimeric and heterodimeric proteins (see, e.g., Pack and Pluckthun, 1992; Chang et al., 1994).

In preferred embodiments of the present invention, the dimerization domains are leucine zipper domains. These leucine zippers are characterized by at least 4 and, preferably, at least 5-7 leucine residues that are spaced periodically at approximately every seventh residue (heptad repeat), with each heptad repeat contributing two turns of the α-helix (3.5 residues/turn). The leucine residues appear to have a special function in coiled-coil dimerization, and form part of the hydrophobic interface between the two α-helices in the coiled-coil. The 40 amino acid leucine zipper domains of the proteins Fos and Jun are preferred examples of leucine zipper dimerization domains These domains each have five leucine residues spaced exactly every seventh residue with a number of hydrophilic residues in the intervening positions (the Fos sequence includes three additional leucines which do not fall in the heptad repeat pattern and which, therefore, are not assumed to contribute to heterodimer formation). Modifications of these domains, or even completely artificial sequences, which maintain the over-all helical nature of these sequences (e.g., which do not include proline or hairpin turns), which preserve the over-all hydrophilicity of the helixes, and which preserve the approximate heptad repeat of leucine residues, may also be employed in accordance with the invention. (Note that the scHLX amphipathic helix of Pack and Pluckthun (1992) was tested in a recombinant HLA-DR2 fusion construct but did not lead to successful heterodimer formation.)

### F. Choice of Immunoglobulin Domains

Human immunoglobulins are divided into five broad classes (IgA, IgD, IgE, IgG and IgM) and any of these may be employed in the MHC-immunoglobulin constructs of the present invention. The basic structures of these molecules are extremely well characterized disulfide-linked homodimers of heavy and light chain heterodimers. Thus, the basic immunoglobulin unit resembles the protein of Figure 2 in which a pair of heavy chains, corresponding to elements 50 and 60, are disulfide linked to each other.

The portions of the two heavy chains which are closely associated with each other, 60 and 60', are referred to as the Fc fragment. In some classes of immunoglobulins (i.e., IgA, IgD, and IgG), there is a hinge region 50 between the Fab and Fc fragments. Finally, within, the heavy immunoglobulin chains, there are regions of great variability and regions of great constancy. Each heavy chain includes. three or four constant domains C_{H}1 through C_{H}4, corresponding essentially to elements 50 and 60 (but not to scale). (See, generally, Kuby, 1994). The immunoglobulin constant domains, as their name implies, are relatively invariant in the human population. On the basis of differences in their constant regions, light chains are broadly classified as either κ or λ and, in humans, λ chains are further divided into four subtypes. Similarly, differences in the constant regions of immunoglobulin heavy chains are the basis of the division of these molecules into five broad classes (α, δ, ε, γ and µ chains in IgA, IgD, IgE, IgG and IgM, respectively). Based on minor differences in amino acid sequences in humans, the γ chains have been further subdivided into four subclasses, and the α chains into two. The amino acid sequences of these various immunoglobulin light and heavy chain constant domains have long been known in the art (see, e.g., Kabat et al., 1979) and will not be reproduced here.

In some preferred embodiments, the MHC-immunoglobulin fusion proteins of the present invention include the Fc regions of either IgG (subtypes 1, 2 or 3) or IgM because these Fc domains are capable of activating the classical complement pathway and, therefore, are more useful in some of the therapeutic methods described below. For utilities in which complement activation is not desired or is irrelevant, however, any of the immunoglobulin isotypes may be employed. In addition, as shown in Figure 3, the IgM isotypes are preferred in some embodiments because they can form pentamers of divalent MHC-IgM fusion proteins.

### G. Expression Systems

The MHC fusion proteins of the present invention may be expressed in any standard protein expression system which allows for proper folding and secretion of the desired molecules, or which allows for their recovery as properly folded molecules from inclusion bodies. As a general matter, eucaryotic expression systems are preferred because they are most likely to produce a high yield of properly folded, glycosylated and disulfide-linked molecules. Mammalian cell lines, especially those which are well characterized for protein expression (e.g., CHO cells, COS cells) or those which are known to secrete properly folded, glycosylated and disulfide linked immunoglobulins (e.g., any mAb producing cell line), may be preferred for some uses. Generally, however, these cell lines express too little protein for therapeutic and commercial applications. Therefore, other eucaryotic expression systems, such as the Pichia pastoris yeast system, described below, may be preferred. In addition, preliminary results have shown high levels of expression of an MHC fusion protein in a Drosophila Schneider cell system. It is well within the abilities and discretion of the skilled artisan, without undue experimentation, to choose an appropriate or favorite expression system.

Similarly, once a design (i.e., primary amino acid sequence) for the MHC fusion proteins of the present invention is chosen, one of ordinary skill in the art can easily design appropriate recombinant DNA constructs which will encode the desired proteins, taking into consideration such factors as codon biases in the chosen host, the need for secretion signal sequences in the host (e.g., an α-mating factor secretory signal for yeast expression), the introduction of proteinase cleavage sites within the signal sequence, and the like. These recombinant DNA constructs may be inserted in-frame into any of a number of expression vectors appropriate to the chosen host. The choice of an appropriate or favorite expression vector is, again, a matter well within the ability and discretion of the skilled practitioner. Preferably, of course, the expression vector will include a strong promoter to drive expression of the recombinant constructs and, optionally, a number of marker genes which will simplify the identification and/or selection of transfotmants.

### H. Uses for Monovalent and Multivalent MHC Class II Fusion Proteins

Particular alleles of the MHC have been associated with a variety of diseases, including multiple sclerosis (MS), rheumatoid arthritis (RA), pemphigus vulgaris (PV) and systemic lupus erythematosus (SLE), and it has been postulated that these diseases are, at least in part, autoimmune in nature. That it, is has been suggested that particular MHC proteins "improperly" recognize processed self peptides presented to T cells in the form of complexes with MHC Class I or Class II molecules. In order to demonstrate this, soluble MHC class II proteins which can be loaded with a single self peptide implicated in the disease can be used. For example, using such MHC-peptide complexes, one can probe lesions in MS patients to determine whether the infiltrating T cells are reactive against a particular self peptide bound to a particular syngeneic MHC molecule. More generally, the MHC Class II fusion proteins of the invention may be used to detect T cells having any defined specificity by constructing an MHC fusion protein loaded with the appropriate MHC binding peptide (covalently or non-covalently joined) and detecting the binding and/or activation of T cell contacted with the fusion protein.

Thus, in one aspect, the present invention provides a method for detecting and/or isolating T cells of a defined MHC-peptide complex specificity comprising contacting a population of T cells with monovalent or multivalent MHC Class II fusion proteins of the invention, as described above, which are loaded with a particular MHC binding peptide and which, therefore, define a particular MHC-peptide complex. The activation or proliferation of the T cells may then be determined and used, with an appropriate control, as an indication of whether the T cell population included T cells specific for the defined MHC-peptide complex. Alternatively, the monovalent or multivalent MHC Class II fusion proteins of the invention, having a defined specificity, may be immobilized on a substrate and a population of T cells may be contacted with the immobilized fusion proteins. After allowing a period of time for the binding, if any, of T cells specific for the defined MHC-peptide complex, unbound cells may be washed away and the presence or absence of bound T cells may be used as an indication of whether the T cell population included T cells specific for the defined MHC-peptide complex. In another embodiment, the monovalent or multivalent MHC Class II fusion proteins of the invention may be contacted with a T cell population and, after allowing a period of time for the binding, if any, of the MHC Class II fusion proteins to T cells specific for the defined MHC-peptide complex, unbound fusion proteins may be washed away and the presence or absence of bound fusion proteins may be used as an indication of whether the T cell population included T cells specific for the defined MHC-peptide complex, In all such embodiments, the labeling of the T cells, fusion proteins, or complexes of the MHC-peptide complex with a reactive T cell receptor with fluorescent, radioactive or other markers is preferred to simplify detection. In particular, fluorescent labels may be used in conjunction with FACS (fluorescence-activated cell sorting) techniques to isolate a desired subpopulation ofT cells with a defined MHC-peptide specificity.

In a particularly preferred embodiment, T cells which are reactive to a specific, defined MHC-peptide complex are detected and isolated as described above, and are then used, preferably after proliferation in vitro, for adoptive immunotherapy. Thus, a population of T cells may be obtained from a host (either the subject to be treated or a syngeneic donor). T cells which are reactive for a particular MHC-peptide complex are then detected and isolated using the methods described above. The cells, preferably after several rounds of proliferation to increase their numbers, are then administered (e.g., intravenously, intraperitoneally) to the subject to confer adoptive immunity. Such a procedure may be of particular utility in stimulating adoptive immunity against weak antigens such as tumor associated antigens.

In another aspect, the present invention provides methods for stimulating or activating T cells, in vitro.

In another aspect of the present invention there is provided a soluble monovalent or multivalent MHC/peptide complex for stimulating or activating T cells reactive to a defined MHC/peptide complex.

As noted above, the present invention provides for the production of soluble Class II MHC fusion proteins for which no soluble counterparts had previously existed. Thus, these soluble Class II MHC fusion proteins, loaded with appropriate MHC binding peptides and defining a specific MHC-peptide complex, may now be contacted with T cells in solution, in vivo or in vitro, to specifically stimulate or activate T cells which are reactive to the defined MHC peptide complex. As noted above, the multivalent Class II MHC fusion proteins of the invention are expected to be particularly potent for these purposes. When conducted in vivo (e.g., when the Class II MHC fusion protein of the invention are administered as a pharmaceutical preparation), this serves as a method of vaccination against the MHC binding peptide when presented in the defined MHC-peptide complex. When used for vaccination purposes against pathogens including the MHC binding peptide, of course, the Class II MHC components of the fusion protein are chosen to syngeneic to the subject being vaccinated.

In another aspect, the monovalent or multivalent Class II MHC fusion proteins of the present invention may be used *in vitro* to kill or anergize T cells reactive to a defined MHC-peptide complex, or to tolerize an individual to a particular MHC-peptide complex.

In another aspect of the present invention there is provided a soluble monovalent or multivalent MHC/peptide complex of the present invention for selectively killing T cells reactive to a defined MHC/peptide complex, wherein said MHC/peptide complex further comprises a covalently bound cytotoxic substance. For example, the Class II MHC fusion proteins may be include Fc regions which activate the complement system and, thereby, cause the destruction of T cells to which they bind. Alternatively, the fusion proteins may be engineered to include a cytotoxic substance attached at, for example, the C-terminus or at some other point which does not interfere with the binding of the MHC-peptide complex to T cell receptors (e.g , anywhere along an Fc domain). Such cytotoxic substances include, for example, genistein, ricin, diphtheria toxins. Pseudomonas toxins, and radioactive isotopes (e.g., ¹²⁵I). High doses of many antigens have a tolerizing effect rather than a stimulating effect. Thus, administration of high doses of a Class II MHC fusion protein of the invention can cause tolerization to the MHC-peptide complex, even when lower doses would cause sensitization (i.e., vaccination or immunization). When the goal is to tolerize an individual to an antigen which is normally presented by the subject's own MHC molecules, the Class II MHC components of the fusion protein are chosen so as to be syngeneic with the host. Such circumstances would include tolerization to the antigens which cause allergic reactions, as well as autoantigens which are implicated in autoimmune disease. In other cases, however, the MHC components may be specifically allogeneic so as to tolerize the subject to an MHC-peptide complex which is foreign. Such circumstances would include tolerization to foreign tissue before or after organ or tissue transplantation in which the donor and recipient are not identical with respect to one or more MHC Class II alleles.

By the term "effective amount," with respect to tolerizing an individual to an MHC-peptide complex, is meant an amount sufficient to render T cells, otherwise specific for the antigenic complex, unresponsive to the MHC-peptide complex. T cells which are unresponsive fail to activate when presented with the antigenic complex for which they are specific. By the term "effective amount," with respect to immunizing an individual to an antigen, is meant an amount sufficient to induce an immune response which results in T cells specific for the antigen. Typical ranges of dosages are from 1 nanogram/kilogram to 100 milligrams/kilogram or even 500 milligrams/kilogram. Effective amounts will vary according to such factors as age, sex and sensitivity to the antigen.

Thus, as an example only, consider the diagnostic and therapeutic utilities of the present invention with respect to MS. The contribution of the MHC to MS susceptibility has been examined in a large number of studies (reviewed in Spielman and Nathenson, 1982; Hillert et al., 1994). These studies demonstrated that susceptibility is associated with the MHC class II region and that particular MHC class II haplotypes confer an increased risk. The strongest association is with the HLA-DR2 haplotype (DRB1*1501); approximately 50 to 60% ofMS patients and 20% of normal subjects carry this haplotype. The DR2 haplotype is most common in MS patients from Western Europe, the U.S. and Canada; the haplotype is also increased among MS patients worldwide. Other MHC class II haplotypes (DR4, DR6) have been associated with susceptibility to MS in particular populations (Italians, Jordanian Arabs); however, these associations are not as strong as the association with DR2 (Marrosu et al., 1988; Kurdi et al., 1977).

HLA-DR2 (encoded by the DRA, DRB1*1501 genes) has been shown to present at least two peptides of human myelin basic protein (residues 85-99 and 148-162) to T cells. The MBP(85-99) peptide bound with high affinity to purified DR2, the affinity of the MBP(148-162) peptide was lower. DR2 transfectants (DRA, DRB1*1501) were found to present these MBP peptides to T cell clones that had been generated from blood lymphocytes of MS patients (Chou et al., 1989; Pette et al., 1990, Martin et al., 1990; Ota et al., 1990; Wucherpfennig et al., 1990; Valli et al., 1993, Wucherpfennig et al., 1994). These studies support the hypothesis that T cells specific for MBP and other myelin antigens are involved in the inflammatory response in MS. Direct identification of such T cells in MS lesions is, however, required to prove this hypothesis and this will require soluble, stable MHC complexes with single peptides, such as those provided by the present invention.

A principal difficulty with using soluble MHC/peptide complexes as probes is that the affinity for the TCR is relatively low. T cells compensate for the relatively low affinity of TCRs for MHC/peptide complexes by the interaction of multiple TCR molecules with MHC/peptide complexes on the surface of antigen presenting cells. Indeed, such dimerization of MHC Class II molecules may be important in T cell activation since HLA-DR1 was found as a dimer in the crystal lattice (Brown et al., 1993). The present invention, by providing multivalent MHC fusion proteins addresses this problem. Classical studies with antibodies and their F(ab) fragments have demonstrated that bivalent/multivalent binding results in a striking increase in the 'functional affinity' (also termed the 'avidity'). IgG molecules and F(ab) fragments bind monovalent antigen in solution with equal affinity; however, the binding to multivalent antigens (i.e. cell surface antigens) is greatly strengthened by the bivalent nature of the IgG molecule (Crothers and Metzger, 1972; Dower et al., 1984; Hornick and Karush, 1972). The 'function affinity' of IgG antibodies was found to be approximately 100-fold greater for bivalent than for monovalent binding; this enhancement factor was even greater for multivalent binding by IgM antibodies (factor of 10³ to 10⁴). Thus, the multivalent MHC fusion proteins of the present invention are expected to have far greater avidity for their cognate TCRs than standard, solubilized MHC proteins.

Last, but most important, purified, soluble MHC fusion proteins loaded with a single antigenic peptide may be used in the process of tolerization. Tolerization may be used in the context of treating standard allergies or in the context of such autoimmune disorders as MS, RA, PV and SLE. For example, studies in the murine experimental autoimmune encephalomyelitis (EAE) model have demonstrated that an autoimmune disease can be treated by the administration of soluble MHC/peptide complexes loaded with the autoantigenic peptide (Sharma et al., 1991). Thus, the present invention, which enables the preparation of purified, soluble, empty MHC fusion proteins which can be loaded with single MHC binding peptides, and which enables the production of MHC-immunoglobulin fusion proteins which include Fc regions which can fix complement, provides several new diagnostic and therapeutic methods.

### III. Examples

DNA constructs. The extracellular domains of DRα (residues 1-191 of DRA*0101) and DRβ (residues 1-198 of DRB1*1501) were expressed as fusions with the 40 amino acid leucine zipper dimerization domains of Fos or Jun, respectively (van Straaten et al., 1983; Angel et al., 1988). The entire extracellular domains, rather than C-terminally truncated domains, were employed because charge-charge interactions between the DRα Glu at position 191 and the DRβ Lys at position 198 are thought to facilitate assembly (Cosson and Bonifacino, 1992) of these molecules. The extracellular domains of DRα and DRβ as well as the Fos and Jun dimerization domains were generated by PCR with primers designed to include a seven amino acid linker (VDGGGGG, residues 199-205 of SEQ ID NO: 2) with a SalI restriction site at the C-terminus of the MHC extracellular domains and at the N-terminus of the Fos or Jun leucine zipper domains. The MHC segments were then joined with the Fos or Jun segments through the SalI restriction site. This linker was included between the DR and leucine zipper segments both to facilitate cloning (through the SalI site) and to allow for greater rotational freedom of the chains (through the poly-Gly sequence). These constructs were reamplified by PCR to permit cloning into the XhoI-EcoRI sites of pPIC9 as in frame fusions with the α-mating factor secretion signal. The in-frame cloning into this vector preserved the Lys-Arg-Glu recognition sequence (cleavage C-terminal to Arg) required for cleavage of the α-mating secretion signal by the KEX2 gene product (Brake, 1990).

The following oligonucleotides were used for the construction: DRα forward primer 5' GTA TCT CTC GAG AAA AGA GAG ATC AAA GAA GAA CAT GTG ATC 3', XhoI site underlined (SEQ ID NO: 5); DRα reverse primer 5' GTC ATA GAA TTC TCA ATG GGC GGC CAG GAT GAA CTC CAG 3', EcoRI site underlined (encodes 3' end of Fos segment, stop codon and EcoRI restriction site) (SEQ ID NO: 6); DRβ forward primer 5' GTA TCT CTC GAG AAA AGA GAG GGG GAC ACC CGA CCA CGT TTC 3', XhoI site underlined (SEQ ID NO: 7); DRβ reverse primer 5' GTC ATA GAA TTC TCA ATG GTT CAT GAC TTT CTG TTT AAG 3' EcoRI site underlined (encodes 3' end of Jun segment, stop codon and EcoRI restriction site) (SEQ ID NO: 8). The resulting PCR products are disclosed as SEQ ID NO: 1 and SEQ ID NO: 3. These PCR products were cloned into the XhoI-EcoRI sites of pPIC9 and were verified by restriction mapping and dideoxy-sequencing.

These constructs were first tested in CHO cells (using the native DR α and β chain signal peptides). CHO transfectants were found to assemble and secrete DR αβ heterodimers indicating that the Fos/Jun leucine zipper promoted the proper assembly of DR2 molecules (data not shown). As described below, however, higher levels of expression were obtained in a yeast expression system employing Pichia pastoris. Recent work has demonstrated that Drosophila Schneider cells give the highest level of protein production (-1 mg/liter, compared to 0.3 mg/liter in Pichia pastoris).

Transformation of Pichia pastoris with DRα and β chain constructs. For protein production, the DRα-Fos and DRβ-Jun constructs were expressed in Pichia pastoris under the control of the alcohol oxidase (AOX1) promoter. Pichia pastoris was chosen because stable transformants can be rapidly generated and screened; in addition, several secreted proteins have been produced at very high levels in this system (Cregg et al., 1993).

To direct expression to the secretory pathway, DRα and β chains were cloned into Pichia pastoris expression vector pPIC9 as in frame fusions with the α-mating factor secretion signal (Brake, 1990) The α-mating factor secretion signal is cleaved by the KEX2 gene product at the sequence Leu-Glu-Lys-Arg-Glu (residues 3-7 of SEQ ID NO: 2 and SEQ ID NO: 4), with the cleavage C-terminal to the Arg residue. Although this design results in the addition of a glutamic acid residue to the N-terminus of the mature DRα and DRβ chains, the N-termini of these chains are located in a manner that this additional residue should not affect the assembly of the heterodimer. Molecules expressed as fusions with the α-mating factor secretion signal were efficiently secreted while usage of the PHO1 secretion signal (vector pHIL-S1, Invitrogen) resulted in little or no secretion. For transformation, the expression cassette of pPIC9 can be excised as a BglII fragment; the cassette carries 5' and 3' sequences of the AOX1 gene to allow for integration into the AOX1 locus as well as the HIS4 gene that allows for selection of transformants in histidine deficient media. Genes integrate into the AOX1 locus by homologous recombination; integration into the AOX1 gene disrupts the gene and leads to slow growth if methanol is the only carbon source (methanol utilization deficient phenotype, Mut^{s}) (Cregg et al., 1987).

Thus, pPIC9 plasmid DNA was purified on CsCI gradients and digested with BglII to release the expression cassette (5' end of AOX1 gene-DRα or DRβ chain construct-polyadenylation signal-HIS4 gene-3' end of the AOX1 gene). Transformations were done by spheroplasting of the GS115 strain (following the procedure provided by Invitrogen, San Diego, CA). Briefly, GS 115 cells were grown to mid-log phase in YPD media and spheroplasts were prepared by limited digestion of the yeast cell wall with zymolase (approximately 70% of spheroplasting) (Cregg et al., 1987). Cells were transfected with 5 mg of DRα and DRβ plasmid DNA and transfectants that expressed the HIS4 gene (present in the pPIC9 expression cassette) were selected on HIS^{*-*} plates. Integration of plasmids into the AOX1 locus was confirmed by replica plating of colonies on minimal media plates with methanol or dextrose as the sole carbon source. Transformants that had integrated the plasmid DNA into the AOX1 locus showed little or no growth on methanol plates due to disruption of the alcohol oxidase gene.

Identification of recombinant colonies. A major advantage of the Pichia pastoris system is that transformants can be readily identified: Integration into the AOX1 locus confers a methanol utilization deficient (Mut^{s}) phenotype that can be determined by comparing the growth of duplicate colonies on plates with methanol or dextrose as the sole carbon source. Mut^{s} colonies obtained after cotransformation of plasmids carrying the DRα and DRβ chain constructs were tested by PCR analysis of genomic DNA for the integration of DRα and β chain genes. 27 of 28 colonies with a Mut^{s} phenotype carried DRα and/or DRβ chain genes; four of these colonies (14.2%) had integrated both genes.

Thus, briefly, integration of DRα and DRβ chain constructs was examined by PCR analysis of genomic DNA isolated from individual Mut^{s} colonies. Replica colonies were transferred into 200 ml of lysis buffer (2.5 M LiCl, 50 mM Tris, pH 8.0, 4% triton X-100, 62 mM EDTA) using a sterile toothpick. Acid washed glass beads and an equal volume of phenol/chloroform (1:1) were added and samples were vigorously vortexed. Following centrifugation, the upper phase was transferred.to a clean tube and genomic DNA was precipitated by addition of 2.5 vol of cold EtOH. Following incubation at -20°C for 20 minutes, the pellet was collected by centrifugation, washed with cold 70% EtOH and air-dried. DNA was resuspended in 40 ml of sterile water and denatured at 94°C for 10 minutes; 10 ml of DNA was used for each PCR reaction. DRα and DRβ chains were amplified by PCR for 35 cycles (94°C 1 min, 55°C 2 min, 72°C 2 min) using the oligonucleotides that had been used to generate the DNA constructs; PCR products were resolved on 1% agarose gels stained with ethidium bromide.

Expression and purification of HLA-DR2 heterodimers. The four transformants that carried both DRα and β chain genes were examined for the expression of DR2 heterodimers. Cells were grown for two days in media containing glycerol as the sole carbon source and were then switched to media containing 0.5% methanol. Supernatants and cell lysates were examined by sandwich ELISA using a mAb specific for the DR αβ heterodimer (mAb L243) for capture and a polyclonal DR antiserum (CHAMP) for detection. DR αβ heterodimer was detected in the cell lysates and supernatants of DR αβ transfectants. Transformants that carried only DRα or DRβ chain genes were used as controls; cell lysates and supernatants from these cells were negative in the assay (Figure 5). These experiments demonstrated that the DR αβ heterodimer was assembled and efficiently secreted. The four Pichia clones showed similar expression levels; this is not surprising since all four transformants had integrated the genes into the AOX1 locus.

For large scale expression, cells were grown in a high density fermenter and DR2 molecules were purified from concentrated supernatants by affinity chromatography with the L243 mAb. The mAb used for purification (L243) binds to the DRα chain but only when properly assembled with the DRβ chain. Affinity purification yielded approximately 300-400 mg of HLA-DR2 per liter of culture. SDS-PAGE revealed two bands, the identity of these bands (upper band DRα, lower band DRβ) and appropriate cleavage of the α-mating factor signal peptide were confirmed by N-terminal sequence analysis following separation of DRα and β chains by SDS-PAGE and transfer to a PVDF membrane.

HPLC gel filtration analysis (Bio-Gel SEC 300 mm x 7.8 mm; flow rate 1 ml/min, PBS pH 6.8) OF 10 µg of HLA-DR2 demonstrated that the recombinant protein eluted as a single symmetric peak and only very small amounts of higher molecular weight aggregates were detected. In contrast, HLA-DR1 expressed in the Baculovirus system was found to aggregate unless these molecules were loaded with a high affinity peptide (Stern and Wiley, 1992). These data demonstrated that the DR2 αβ heterodimer was assembled and secreted in the Pichia pastoris expression system even in the absence of a high affinity peptide. Importantly, the purified molecules did not aggregate even though they had not been loaded with a high affinity peptide.

Specifically, induction of high density cultures was carried out using a Inceltech LH series fermenter equipped with monitors and controls for pH, dissolved O₂, agitation, temperature, and air flow. A 100 ml YNB-glycerol overnight culture was used to inoculate the fermenter which contained 10 liters of fermentation basal salts medium (0.93 g/L calcium sulfate 2 H₂O, 18.2 g/L potassium sulfate, 14.9 g/L magnesium sulfate 7 H₂O, and 6.5 g/L potassium hydroxide) containing 4% glycerol (w/v) plus 43.5 ml of PTM₁ trace salts (24 mM CuSO4, 0.53 mM NaI, 19.87 mM MnSO₄, 0.83 mM Na₂MoO₄, 0.32 mM boric acid, 2.1 mM CoCl₂, 0.15 mM ZnCl₂, 0.23 mM FeSO₄, and 0.82 mM biotin) at 30°C. Dissolved O₂ was maintained above 20% by adjusting aeration and agitation, and pH was maintained at 6.0 by the addition of 28% (v/v) ammonium hydroxide. Growth was continued until the glycerol was exhausted (20 hours). A glycerol fed-batch phase was initiated by the limited addition of 50% (w/v) glycerol and 12 ml PTM₁ salts per liter of glycerol at 18.15 ml/hr/L initial fermentation volume until the culture reached a wet cell weight (wcw) of 200 g/L (22 hours). After the glycerol fed-batch phase, the culture was induced by replacing the glycerol feed with a methanol-batch feed (100% methanol containing 12 ml PTM₁ trace salts per liter of methanol) at 1 ml/hr/L. The methanol feed was gradually increased in 10% increments every 30 minutes to a rate of 3 ml/hr/L and the fermentation continued for a duration of 96 hours.

Supernatants were concentrated by ultrafiltration on a YM30 membrane (Amicon) and passed over an anti-DR (mAb L243) affinity column at a flow rate of approximately 10 ml/hour. Following extensive washing with PBS, heterodimers were eluted with 50 mM glycine, pH 11.5. Eluates were immediately neutralized by addition of 2 M Tris, pH 8.0, dialyzed against PBS and concentrated by ultrafiltration. Protein concentrations were determined by Coomassie Plus Protein Assay (Pierce, Rockford, IL) using bovine serum albumin as a standard.

Loading of soluble HLA-DR2 molecules with a high affinity peptide. A human myelin basic protein (residues 85-99) that is recognized by DR2 restricted T cell clones from MS patients was previously shown to bind with high affinity (IC₅₀ of 4.2 nM) to detergent soluble DR2 purified from L cell transfectants (Wucherpfennig et al., 1994 and 1995a). A biotinylated peptide with an SGSG linker between the biotin moiety and the MBP sequence (i.e., biotin-SGSG-MBP(85-99)) was used to examine the specificity of peptide binding to recombinant DR2. Peptide binding was assessed by incubating DR2 molecules (50-400 nM) with the biotinylated peptide (2 µM) at 37°C for different periods of time; non-biotinylated peptide was used as a competitor to demonstrate the specificity of binding (Figure 6). DR2/peptide complexes were then captured on an ELISA plate using the L243 mAb and the amount of bound biotinylated peptide was quantitated using peroxidase-labeled streptavidin and ABTS as a peroxidase substrate (detection at 405 nm).

Peptide binding to DR2 was strongly dependent on the pH, with a maximum observed at pH 7 to pH 8; relatively little binding was observed at pH 5. A similar pH optimum had previously been observed for binding of the MBP peptide to detergent soluble DR2 (Wucherpfennig et al., 1994). Binding of peptide was dependent on the relative molar ratio of DR versus peptide, with a maximum of binding at a 10-fold molar excess of peptide over DR2 (Figure 6). Binding was specific because it could be blocked by an excess of non-biotinylated MBP(85-99) peptide but not by an analog peptide Val89→Asp in which the P1 anchor residue (Val 89) of MBP(85-99) had been substituted by aspartic acid (Figure 6).

To determine what fraction of recombinant molecules could be loaded with a single peptide, complexes of DR2 and the biotinylated MBP peptide were precipitated with streptavidin beads. Following precipitation, DRα and β chains were resolved by SDS-PAGE and detected by Western blotting using a polyclonal DR antiserum. Approximately 50% of the molecules were precipitated with streptavidin beads and 50% remained in the supernatant. Control experiments demonstrated that precipitation of the DR2/peptide complexes was specific as the molecules were not precipitated when control agarose beads, an unlabeled MBP peptide or an excess of unlabeled peptide over biotinylated peptide were used; rather the DR2 molecules remained in the unbound fraction.

For immunoprecipitation experiments, DR2 (400 nM) was incubated with biotinylated peptide (2 µM) in a 50 ml volume in PBS, 1 mM EDTA, 1 mM PMSF, pH 7.2 for 24 hours at 37°C. DR2-peptide complexes were precipitated with streptavidin-agarose beads. Beads were first blocked with 3% bovine serum albumin in PBS, 0.1% NP40 for 1 hour at 4°C; beads were then pelleted and the DR2/peptide samples added. Following a 1 hour incubation, beads were washed three times with blocking buffer. DR2-peptide complexes were eluted from streptavidin beads by heating in 1xSDS-PAGE buffer at 94°C for 3 minutes. Samples were resolved on a 12.5% SDS-PAGE and transferred to immobilon membrane (Millipore). Blots were blocked overnight with 5% non-fat dry milk in 50 mM Tris, pH 8.0, 150 mM NaCl, 0.2% Tween 20 (TBST buffer). Precipitated DR α and β chains were detected with a polyclonal DR antiserum (CHAMP, 1:50,000 in blocking buffer for 90 min). Blots were washed in TBST buffer and incubated for 30 min with a peroxidase conjugated anti-rabbit IgG antibody (1:10,000 in blocking buffer). Following extensive washing in TBST, bands were detected by enhanced chemiluminescence (Amersham, Arlington Heights, IL).

In a separate set of experiments, peptide binding to recombinant DR2 was quantitated by capturing DR2 molecules to ELISA plates with an immobilized DR antibody. Standard binding conditions were 37°C for 24 hours in PBS, pH 7.2, 1 mM EDTA, 1 mM PMSF. Following peptide binding, bound peptide was quantitated by ELISA. Plates were coated with 200 ng/well of purified L234 in 0.1 M bicarbonate, pH 9.6 overnight at 4°C. Non-specific binding sites were blocked with 3% BSA in PBS, 0.05% Tween 20 for 2 hours. Samples were diluted in blocking buffer and added to the wells (1 hour). HLA-DR2 bound biotinylated peptide was quantitated with streptavidin-peroxidase using ABTS as a peroxidase substrate; absorbance was read at 405 nm.

Kinetics of peptide binding to soluble HLA-DR2 molecules. The kinetics of peptide binding by detergent soluble DR2 purified from an EBV transformed B cell line (Gorga et al., 1987) and by recombinant DR2 were compared (Figure 7). Equimolar amounts of both DR2 preparations (200 nM) were incubated with the biotinylated MBP peptide 2 µM) at 37°C for different periods of time; the amount of DR-bound peptide was examined by ELISA using the DR specific L243 mAb antibody for capture and streptavidin-peroxidase for detection of DR-bound peptide. The kinetics of peptide binding were strikingly different: With recombinant molecules, the kinetics of binding were much faster and a much larger fraction of the molecules were loaded (50% maximum binding after only 3 hours with a plateau after 18 hours). In contrast, the kinetics of peptide binding to DR2 from B cells were slow; the faction of peptide loaded molecules slowly increased over a 48 hour period without reaching a plateau (Figure 7). These results are explained by the fact that the majority of DR molecules purified from B cells are already occupied with high affinity peptides, as demonstrated by peptide elution studies and crystallization of HLA-DRI (Chicz et al., 1993; Brown et al., 1993). In contrast, the peptide binding site of a large fraction of the recombinant DR2 molecules is empty and readily available for binding by a high affinity peptide.

Expression of the DR2-IgG fusion protein. DR2-IgG fusion proteins were expressed in the Drosophila Schneider cell system. The DR2-IgG design was chosen both to increase the affinity for the T cell receptor, and to attach an effector domain, the Fc region of IgG2a. Complement fixation may result in the lysis of target T cells following binding of DR2-IgG molecules to the T cell receptor. DR2-IgG molecules may therefore be useful for the selective depletion of autoaggressive T cells. Studies that compared the affinity of bivalent IgG antibodies and of their monovalent F(ab) fragments have shown that the bivalent nature of IgG greatly increases the functional affinity for antigen (Crothers and Metzger, 1972; Dower et al, 1984; Hornick and Karush, 1972).

cDNA constructs for DR2-IgG fusion molecules. Bivalent DR2 fusion proteins were expressed by fusing the Fc part of IgG2a to the 3' end of the DRα-Fos cDNA construct described above. In this design, the DRα-Fc chain corresponds to the antibody heavy chain and the DRβ-Jun construct to the antibody light chain.

The Fc part of IgG2a was amplified by RT-PCR from a mouse hybridoma (L243) that secretes a IgG2a mAb. The PCR product was fused in frame with the DRα-Fos construct by overlapping PCR. Overlapping PCR was done with a primer for the Fc part that overlapped by 20 bp with the 3' end of the DRα-Fos construct. DRα-Fos and Fc were amplified separately, gel purified, mixed and amplified using oligos representing the 5' end of DRα and the 3' end of IgG2a. The construct was cloned into the EcoRI-BamHI sites of the pRmHa-3 expression vector under the control of the metallothionein promoter. The insert was checked by restriction mapping and dideoxy-sequencing.

### Production of DR2-IgG fusion molecules

The Drosophila Schneider cell system was chosen for the expression of the DR2-IgG fusion protein for the following reasons: (1) Recombinant antibodies have previously been expressed in insect cells (Gauthier et al., submitted for publication), (2) In the pRmHa-3 expression vector, genes are under the control of the strongly inducible metallothionein promoter, (3) Schneider cells can be grown to a high cell density in serum free media, and (4) Large scale production of protein is more straightforward than in another insect cell system (the Baculovirus system) since stable transfectants are generated.

Stable transfectants were generated by the contransfecting Schneider cells with the DRα-IgG and DRβ chains vectors as well as with plasmid pH8CO. This vector confers resistance to selection by methotrexate. Transfectants were selected with 0.1 µM methotrexate in Schneider media, 10% fetal calf serum. Transfectants were cloned by limiting dilution, and the secretion of DR2-IgG molecules was examined by ELISA using antibodies specific for the Fc segment of IgG as well as the L243 mAb specific for the DRαβ heterodimer.

Transfectants were grow to a density of ∼10x10⁶/ml and expression was induced by adding CuSO₄ to a final concentration of 1mM. Supernatants were harvested five days following induction and concentrated by ultrafiltration. DR2-IgG molecules were purified by affinity chromatography using mAb L243. Purity was examined by SDS-PAGE; for comparison, purified mouse IgG was also run on the gel. Western blot analysis with a polyclonal antiserum confirmed the identity of the two bands. Peptide binding experiments demonstrated that DR2-IgG molecules were properly folded and functional.

Expression of DR2-tag fusion molecules for the generation of DR2-tetramers. Biotin ligase specifically biotinylates a lysine residue within a 14-amino acid recognition sequence (LGGIFEAMKMELRD, SEQ ID NO: 9) (Shatz, 1993) and, therefore, a DNA sequence encoding this "tag" was added to the DRα-Fos construct. This "DRα-Fos-tag" construct was cloned into the EcoRI and Sall sites of Drosophila expression vector pRmHa-3 under the control of the inducible metallothionein promoter. Drosophila Schneider cells stably co-transfected with the DRα-Fos-tag and DRβ-Jun constructs were generated as described above for DR2-IgG. The resulting "DR2-tag" fusion molecules differ from the DR2-Fos/Jun fusion proteins only by the addition of the biotinylation sequence tag to the C-terminus of the DRα-Fos construct and the DR2-tag molecules were affinity purified from supernatants using mAb L243 as described above.

Site specific biotinylation of these DR2-tag molecules allows the assembly ofDR2-tag-biotin tetramers on streptavidin because streptavidin has four biotin binding sites. Thus, tetramers are made by mixing the DR2-tag-biotin molecules and streptavidin at a 4:1 molar ratio. The DR2-tag-biotin/streptavidin tetramer does not carry an effector domain but can be conjugated to a toxin.

Expression of biotin ligase for the biotinylation of DR2-tag molecules. Biotin ligase was over-expressed in E. coli; the biotin ligase cDNA (provided by S. Lesley, Promega Corporation) was cloned as a NdelI-xhoI fragment into the procaryotic expression vector pET22b under the control of the T7 promoter. This construct was transfected into E. coli strain BL21/DE3 which is lysogenic for the T7 RNA polymerase gene under the control of the lacZ promoter. Protein expression was induced by addition of IPTG to ImM for 4 hours. Cells were then harvested by centrifugation, resuspended in 20 mM Tris, pH 8.0, 100 mM NaCl. Cells were sonicated and insoluble material was removed by centrifugation, yielding 5 ml of a soluble cytoplasmic protein fraction from 100 ml of culture.

Biotinylation was performed at 37°C in a 100 µl volume with 0.1 to 10µl of enzyme, 1 mM of ATP and 1 or 10 µM of biotin. Following the reaction, recombinant DR2-tag-biotin molecules were captured on a 96-well plate coated with the L243 mAb and the degree of biotinylation was quantitated using peroxidase conjugated streptavidin.

Western blot analysis of the biotinylated α chain of DR2-tag. DR2-tag molecules were biotinylated with biotin ligase in the presence of ATP and biotin. A Western blot was sequentially probed with a polyclonal DR antiserum and with streptavidin peroxidase. This experiment demonstrated specific biotinylation of the DRα chain (which carries the 14-amino acid biotin ligase recognition sequence) by biotin ligase.

Generation of DR2-tetramers. Fluorescein-labeled streptavidin was used to examine the formation of DR2-tag-biotin tetramers. Fluorescein absorbs at 492 nm, allowing detection during HLPC gel filtration chromatography (Bio-Gel SEC 300 mm x 7.8 mm; flow rate 1 ml/min, PBS pH 6.8). Streptavidin (MW 60 kDa) eluted as a single peak at 8.3 minutes on the HPLC gel filtration column. The streptavidin-DR2-tag-biotin complex eluted at 5.8 minutes. Intermediates with one, two or three DR2 fusion molecules bound to streptavidin were observed when smaller amounts of DR2-tag-biotin were used for complex formation. MW standards confirmed the predicted molecular weight of streptavidin and the streptavidin-DR complex.

Generation of DR2/peptide complexes loaded with a single peptide. A (His)₆-tagged MBP(85-99) peptide was used to purify DR2 fusion proteins loaded with a single peptide by metal affinity chromatography. DR2-tag molecules were incubated with the (His)₆-tagged MBP peptide and precipitated with the metal affinity resin (Talon Metal Affinity Resin, Clontech). DR2/peptide complexes were eluted under mild conditions (1 mM EDTA). Eluted DR2 molecules were analyzed by Western blot analysis, using a polyclonal DR antiserum for detection These experiments demonstrated that defined DR2/peptide complexes can be generated at a yield of ∼50%.

Binding of T cells to DR2/peptide complexes. The binding of T cell receptors on the surface of human T cell clones to recombinant DR2/peptide complexes was examined. Biotinylated DR2-tag molecules were loaded with the MBP(85-99) peptide and captured on a streptavidin coated plate; the binding of fluorescent-labeled T cells to immobilized DR2/peptide complexes was quantified. As a positive control, wells were coated with an anti-CD3 mAb.

Binding was examined using a human DR2 restricted T cell clone (Ob. 1A12) specific for MBP(85-99) and a DR4 restricted control clone (Go.P3.1) specific for residues 190-204 of human desmoglein 3 protein. Specific binding to DR2/MBP(85-99) complexes was only observed with the MBP(85-99) specific T cell clone. Furthermore, binding was observed only with the DR2/MBP(85-99) complex, and not with empty DR2.

Binding was examined by capturing biotinylated DR2-tag molecules on a streptavidin coated plate. Non-specific binding sites were blocked with 0.1% BSA in PBS. T cells were labeled with BCEFC-AM, a fluorescent membrane probe, for 30 minutes at 37°C, washed and added to the plate for 20 minutes at 37°C. Following three washes, the fraction of T cells that bound to DR2/peptide complexes or to the anti-CD3 mAb was determined in a fluorescent plate reader.

Production of DR2-IgM Fusion Molecules. DR2-IgM molecules carry ten DR2/peptide arms (there are five IgM monomers in the pentamer; each IgM monomer has two arms, like IgG). Since DR2-IgG molecules have two DR2/peptide arms, the functional affinity of DR2-IgM molecules for the T cell receptor is expected to be much higher. A significant increase in affinity would improve the sensitivity for immunohistochemical staining of MS lesions as well as the therapeutic effectiveness of these molecules. IgG antibodies are expressed at high levels by insect cells (Potter et al., 1993; Gauthier et al., submitted for publication). IgM antibodies have been expressed in mammalian cells (CHO cells), but expression of IgM antibodies in insect cells has not yet been reported. DR2-IgM molecules may be particularly useful for immunotherapy for the following reasons: (1) A high affinity for the T cell receptor on target T cells, (2) Complement fixation by the Fc segment of IgM, and (3) Longer serum half life.

The Fc segment of murine IgM is fused in frame to the 3' end of the DRα-Fos segment, as previously described for the DRα-IgG construct. The DRα-IgM construct is cloned into, for example, the EcoRI-BamHI sites of the pRmHa-3 expression vector, under the control of the inducible metallothionein promoter (Bunch et al., 1988). The DRα-IgM and DRβ chain fusion constructs are cotransfected with a gene encoding the murine J-chain. The J-chain facilitates assembly and secretion of IgM molecules by mammalian cells (Matsuuchi et al., 1986). The murine J-chain may be cloned into, for example, expression vector pUC-hygMT which confers resistance to hygromycin. Stable transfectants may then be selected using hygromycin at 100 µg/ml in Schneider cell media (Sigma) supplemented with 10% insect cell tested fetal calf serum. Transfectants are cloned by limiting dilution and tested for expression of DR2-IgM molecules following induction with CuSO₄.

Secretion of DR2-IgM molecules may be assessed by immunoprecipitation with mAb L243, followed by Western blot analysis with antibodies specific for the Fc segment of murine IgM. For protein production, transfectants can be adapted to serum free media (ExCell 400, JRH Biosciences).

These constructs also can be transfected into CHO cells or into a murine B cell line (M12.C3). CHO cells were previously shown to secrete recombinant IgM antibodies at high levels and have been used for the expression of a CD2-IgM fusion protein (Wood et al., 1990; Arulanandam et al., 1993). For expression in these cells lines, the DRα-IgM and DRβ chain constructs can be cloned into eucaryotic expression vectors. The DRα-IgM construct can be cloned into, for example, pcDNA3, which carries the neomycin resistance gene, the DRβ-Jun construct can be cloned in the pcDNAI vector (Invitrogen). Cells can be transfected by electroporation and stable transfectants can be selected with G418. Secretion of DR2-IgM molecules can be assessed by immunoprecipitation with mAb L243 and by Western blot analysis.

Use of DR2-Ig fusion proteins for the selective depletion of T cells. DR2-Ig fusions proteins may be useful for the selective depletion of T cells that recognize DR2 bound self-peptides. Binding of DR2-Ig fusion proteins by the T cell receptor may lead to complement fixation and lysis of target T cells. Multivalent DR2 molecules could also be conjugated to genistein, a tyrosine kinase inhibitor that induces apoptosis following uptake by target cells.

Affinity of multivalent DR2/peptide complexes for the T cell receptor. The binding of multivalent DR2/peptide complexes to the TCR will be examined using human DR2 restricted T cell clones. DR2 molecules will be loaded with the MBP(85-99) peptide and labeled with [²⁵I] using immobilized chloramine T (Iodobeads, Pierce). In the binding assay, a fixed number of T cells (1x10⁶ cells, 1 ml) will be incubated with 6-10 different concentrations of radiolabeled DR2/peptide complexes in PBS, 1.0% BSA, 0.02% NaN₃. Radiolabeled molecules will be used at concentrations at which only a small fraction (less than 10%) of TCRs on target cells will be occupied.

First, the incubation time required to reach equilibrium will be determined; cell-bound and unbound DR2/peptide complexes will be separated by rapid (10-15 sec.) centrifugation through a layer of 84% silicone (d=1.050), 16% paraffin oil (d=0.838). Cell bound radioactivity will be quantitated in a γ-counter and data will be analyzed on Scatchard plots to determine K (dissociation constant) and n (number of TCR molecules on target cells). Several of controls will be included to demonstrate specificity of binding: (1) T cell clones with an unrelated MHC/peptide specificity, (2) DR2/peptide complexes that were loaded with control peptides, and (3) Competition of binding by an excess of unlabeled DR2/peptide complexes.

It will be of particular interest to determine the kinetics of binding by monovalent (DR2), bivalent (DR2-IgG) and multivalent (DR2-IgM, DR2-tetramers) molecules to the TCR. "On" rates will be determined by incubating target cells with radiolabeled ligands for different time periods at 37°C (in the presence of 0.02% sodium azide to prevent internalization of the TCR), followed by rapid separation of reactants. "Off" rates will be determined by incubating T cells with labeled ligands until equilibrium is reached. Cells will then be washed, incubated for different periods of time and the amount of cell bound radioactivity will be determined. Off rates are expected to be significantly different for monovalent, bivalent and multivalent ligands. Classical studies with IgM antibodies have shown that multivalent attachment dramatically slows the dissociation of bound antibody (Crothers and Metzger, 1972; Hornick and Karush, 1972).

Complement mediated lysis of T cells specific for DR2-Ig fusion proteins. The Fc segment of IgG2a was chosen for the DR2-IgG fusion protein because IgG2a fixes complement. IgM also fixes complement, allowing complement mediated lysis of target T cells by fusion proteins to be assessed. T cells will be incubated with DR2-IgG or DR2-IgM complexes; cells will then be washed and incubated with rabbit serum complement diluted in media (1:5 to 1:20 dilution). Rabbit serum complement will be obtained form Cedarlane Laboratories and will be pretested to ensure that the lot does not have nonspecific cytotoxicity against human T cells; complement will be aliquoted and stored at -70°C. Cytotoxicity will be determined after 30 and 60 minutes of incubation at 37°C by trypan blue staining (% cytotoxicity = [number of dead cells /number of live + dead cells] x 100). A mAb specific for human CD3 (OKT3, IgG2a) that fixes complement will be used as a positive control. Specificity of lysis will be assessed using control T cell clones as well as DR2 molecules loaded with control peptides.

Coupling of DR2/peptide complexes to toxins that induce apoptosis. Multivalent DR2 molecules of all three designs, DR2-IgG, DR2-IgM and DR2-tetramers. will be conjugated to toxin moieties as another means of mediating selective T cell death. Genistein, a tyrosine kinase inhibitor, may be particularly effective for this purpose. In a recent study, genistein coupled to CD 19 mAb was found to be highly effective in eradicating a human B cell leukemia from SCID mice (Uckun et al., 1995). A single dose of 25 µg of a genistein-mAb conjugate provided complete protection from a lethal challenge with the B cell leukemia. CD19 is a B lineage specific surface molecule; the antibody conjugate was shown to induce apoptosis following internalization by receptor mediated endocytosis. T cell receptors are endocytosed following recognition of DR2/peptide complexes (Valitutti et al., 1995); it is therefore likely that multivalent DR2/peptide complexes will be taken up target T cells following binding to the T cell receptor.

Genistein will be conjugated in multivalent DR2/peptide complexes by photoaffinity crosslinking using a photosensitive 18.2 Å long non-cleavable hetero-bifunctional crosslinking agent (Sulfo-SANPAH) as described by Uckun et aL (1995). The DR2-toxin conjugates will be tested using the human DR2 restricted T cell clones. T cells will be incubated with the DR2-toxin conjugates and the induction of apoptosis will be assessed by agarose gel electrophoresis of genomic DNA. Nucleosomal fragmentation of DNA will be examined by ethidium bromide staining. DR2 molecules loaded with control peptides as well as control T cell clones will be used to demonstrate the specificity of apoptosis induction.

Apoptosis induction by DR2-toxin conjugates will be quantitated by flow cytometry following end labeling of fragmented DNA ends (TUNEL procedure). The free ends of nuclear DNA fragments will be labeled with dioxygenin-conjugated nucleotides, using the enzyme terminal deoxynucleotidyl transferase (TdT). Cells will be fixed and permeabilized by treatment with 70% EtOH. The 3'-OH ends of nuclear DNA fragments will be labeled with dioxygenin-dUTP, dioxygenin-dATP and TdT, followed by detection of labeled DNA ends with a fluorescein labeled anti-dioxygenin antibody (ApopTag, in situ apoptosis detection kit, Oncor). FACS analysis will be used to determine the fraction of cells that have undergone apoptosis. Cells grown for 12 hours at a low serum concentration (1% serum) will be used as a positive control. Specificity of apoptosis induction will be demonstrated by using control T cells clones and DR2 molecules loaded with control peptides.

### T cell binding to immobilized DR2/peptide complexes.

Previous studies had demonstrated that recombinant, soluble DR2 molecules specifically bind peptides. To examine if recombinant DR2/peptide complexes are recognized by T cell receptors, T cell adhesion assays were performed using biotinylated DR2/peptide complexes that were captured on streptavidin coated microtiter plates. MBP(85-99) specific T cell clones and control T cell clones were labeled with BCEFC-AM, a fluorescent membrane probe, washed and incubated for 30 minutes at 37°C with immobilized DR2/peptide complexes. Following washing, the fraction of bound T cells was determined in a fluorometer. Binding of MBP(85-99) specific, DR2 restricted T cells was only observed when DR2/MBP(85-99) complexes were used, but not when DR2 molecules were loaded with a control peptide. Also, a single amino acid substitution at a primary TCR contact residue in the peptide abolished T cell binding. Binding to DR2/MBP(85-99) complexes was not observed with control T cell clones.

### DR2-Ig fusion proteins: Expression of DR2 with a covalently linked MBP peptide.

DR2-Ig fusion proteins are generated to allow multivalent binding to TCRs on target T cells (2 DR2/peptide arms in the DR2-IgG fusion protein, 10 DR2/peptide arms in the DR2-IgM fusion protein). In order to ensure that all binding sites in these molecules will be loaded with the same peptide, DR2 molecules were expressed with a covalently linked MBP peptide. The MBP(85-99) sequence was attached to the N-terminus of the mature DR β chain through a 16-amino acid linker (linker sequence: SGGGSLVPRGSGGGGS, SEQ ID NO: 10). This cDNA construct was used to express DR2 molecules and DR2-IgG molecules with a linked MBP peptide in Drosophila Schneider cells.

### DR2-Ig fusion proteins: Expression of DR2-IgM fusion protein.

The DR2-IgM fusion protein may be of greatest utility for the depletion of antigen specific T cells in patients with MS since they have the highest valency. DR2-IgM fusion proteins were not secreted by Drosophila Schneider cells; expression in COS cells was therefore performed. DR2-IgM fusion molecules were secreted when COS cells were transfected with the cDNA constructs.

### DR2 molecules with site-specific biotinylation.

The 14-amino acid recognition sequence for biotin ligase was attached to the C-terminus of the DR α chain; biotin ligase specifically biotinylates a lysine residue within this 14-amino acid recognition sequence (LGGIFEAMKMELRD, SEQ ID NO: 9) (Schatz, 1993). This construct was cotransfected with the DR β chain cDNA construct into Drosophila Schneider cells and stable transfectants were generated. DR2-biotin tag molecules were affinity purified and site-specific biotinylation was performed with biotin ligase expressed in E. coli. Site specific biotinylation was demonstrated by Western blot analysis; probing with a DR antiserum labeled both DR α and DR β chains while probing with streptavidin only labeled the biotinylated DR α chain.

### Staining of T cells with DR2/peptide complexes immobilized on fluorescent microbeads.

DR2-biotin tag molecules were used to generate highly multivalent probes for the specific staining of antigen specific T cells. DR2-biotin tag molecules were bound to small, highly fluorescent microspheres (40 nm in diameter) to which streptavidin had been conjugated. Staining of antigen specific T cells was examined by FACS. Staining was only observed with MBP(85-99) specific T cell clones but not with control clones; the staining intensity was similar to that observed with a CD4 antibody. Staining was highly specific since a single amino acid substitution in the MBP peptide at a TCR contact residue abolished staining of MBP specific T cell clones.

### Expression of DQ8 molecules associated with insulin dependent diabetes.

The leucine zipper dimerization domains of Fos and Jun were also used to express soluble DQ molecules (DQ1 and DQ8 which are associated with susceptibility to pemphigus vulgaris and insulin dependent diabetes, respectively). The same design was used as described above for recombinant DR2 (including splice points). Stable transfectants were generated using Drosophila Schneider cells and soluble DQ molecules were affinity purified. Peptide binding studies using peptides that were previously shown to bind to DQ1 or DQ8 demonstrated that the molecules were functional.

### References

Acha-Orbea et al. (1988) Cell 54:263-273.

Angel et al. (1988) Nature 332:166-171.

Arulanandam et al. (1993) J. Exp. Med. 177:1439-1450.

Avva and Cresswell (1994) Immunity 1:763-774.

Bjorkman et al. (1987) Nature 329:512-5 18.

Bodmer et al. (1995) Tissue Antigens 46:1-18.

Brake (1990) Meth Enzymol. 185:408-421.

Brown et al. (1993) Nature 364:33-39.

Bunch et al. (1988) Nucl. Acid Res. 16:1043-1061.

Chang et al. (1994) Proc. Natl. Acad. Sci. (USA) 91:11408-11412.

Chicz et al. (1992) Nature 358:764-768.

Chicz et al. (1993) J. Exp. Med. 178:27-47.

Chou et al. (1989) J. Neurosci. Res. 23:207-216.

Corr et al. (1994) Science 265:946-949.

Cosson and Bonifacino (1992) Science 258:659-662.

Cregg et al. (1987) Bio/Technology 5:479-485.

Crothers and Metzger (1972) Immunochemistry 9:341-357.

Davies et al. (1994) Nature 371:130-136.

Dower et al. (1984) J. Immunol. 132:751-758.

Ebers (1996) IBC's Fourth Annual International Conference: Advances in the Understanding and Treatment of Multiple Sclerosis. Meeting Abstract, San Francisco June 1996.

Ferré-D'Amaré et al. (1993) Nature 363:38-45.

Gorga et al. (1987) J. Biol. Chem. 262:16087-16094.

Grégiore et al. (1991) Proc. Natl. Acad. Sci. (USA) 88:8077-8081.

Hammer et al. (1994) J. Exp. Med. 180:2353-2358.

Hammer et al. (1995) J. Exp. Med. 181:1847-1855.

Hauser (1996) IBC's Fourth Annual International Conference: Advances in the Understanding and Treatment of Multiple Sclerosis. Meeting Abstract, San Francisco June 1996.

Heldin (1995) Cell 80:213-223.

Hilbert et al. (1994) J. Neuroimmunol. 50:95-100.

Holz et al. (1996) J. Neurosci. 16:467-477.

Hornick and Karush (1972) Immunochemistry 9:325-340.

Hu et al. (1990) Science 250:1400-1403.

Jardetzky et al. (1996) Proc Natl. Acad. Sci. (USA) 93:734-738.

Kabat et al. (1979) "Sequences of Immunoglobulin Chains: Tabulation and Analysis of Amino Acid Sequences of Precursors, V-Regions, C-Regions, J-Chain and β₂-Microglobulins," NIH Publication No. 80-2008, U.S. Dept. of Health Education and Welfare.

Kuby (1994) Immunology, 2nd Edition, W.H. Freeman and Company, New York.

Leonard et al. (1984) Nature 311:626-631.

Manolios et al. (1990) Science 249:274-277.

Marrosu et al. (1988) Neurology 38:1749-1753.

Marsh and Bodmer (1995) Tissue Antigens 45:258-280.

Matsui et al. (1991) Science 254:1788-1791.

Matsuuchi et al. (1986) Proc. Natl. Acad. Sci. (USA) 83:456-460.

Oas and Endow (1994) TIBS 19:51-54.

O'Shea et al. (1989) Science 245:646-648.

O'Shea et al. (1991) Science 254:539-544.

Pack and Pluckthun (1992) Biochemistry 31:1579-1584.

Pette et al. (1990) Proc Natl. Acad. Sci. (USA) 87:7968-7972.

Potter et al. (1993) Int. Rev. Immunol 10:103-112.

Sato et al. (1989) Biochem. Biophys Res. Comm. 163:1473-1480.

Schatz (1993) Bio/Technology 11:1138-1143.

Sharma et al. (1991) Proc. Natl. Acad. Sci. (USA) 88:11465-11469.

Spielman and Nathenson (1982) Epidemiol. Rev. 4:45-65.

Steinman (1996) Cell 85:299-302.

Stern and Wiley (1992) Cell 68:465-477.

Stern et al. (1994) Nature 368:215-221.

Strominger and Wiley (1995) JAMA 274:1074-1076.

Suda et al. (1993) Cell 75:1169-1178.

Sykulev et al. (1994) Proc. Natl. Acad. Sci. (USA) 91:11487-11491.

Todd et al. (1988) Science 240:1003-1009.

Uckun et al. (1995) Science 267:886-891.

Valitutti et al. (1995) Nature 375:148-151.

Valli et al. (1993) J. Clin. Invest. 91:616-628.

van Straaten et al. (1983) Proc. Natl. Acad. Sci. (USA) 80:3183-3187.

Viskochil et al. (1991) Mol Cell Biol. 11:906-912.

Weber et al. (1992) Nature 356:793-796.

Wettstein et al. (1991) J. Exp. Med. 174:219-228.

Wood et al. (1990) J. Immunol. 145:3011-3016.

Wucherpfennig and Strominger (1995a) Cell 80:695-705.

Wucherpfennig and Strominger (1995b) J. Exp. Med. 181:1597-1601.

Wucherpfennig et al. (1991) Immunol. Today 12:277-282.

Wucherpfennig et al. (1994a) J. Exp. Med. 179:279-290.

Wucherpfennig et al. (1994b) J. Immunol. 150:5581-5592.

Wucherpfennig et al. (1995) Proc. Natl. Acad. Sci. (USA) 92:8896-8900.

## Claims

1. A soluble Class II Major Histocompatibility Complex fusion protein comprising:
a first polypeptide comprising a fusion of, toward the N-terminus, at least an MHC Class II binding domain of a MHC Class II α chain and, toward the C-terminus, a first coiled-coil dimerization domain; and
a second polypeptide comprising a fusion of, toward the N-terminus, at least an MHC Class II binding domain of a MHC Class II β chain and, toward the C-terminus, a second coiled-coil dimerization domain;
wherein said first and second polypeptides associate under physiological conditions to form a heterodimeric MHC class II molecule that is capable of selectively binding an MHC binding peptide to form a MHC/peptide complex.

2. A soluble Class II Major Histocompatibility Complex fusion protein as claimed in claim 1, wherein said MHC Class II binding domain comprises an extracellular domain of an MHC Class II alpha chain.

3. A soluble Class II Major Histocompatibility Complex fusion protein as claimed in claim 2, wherein said extracellular domain comprises residues 5-180 of an MHC Class II alpha chain.

4. A soluble Class II Major Histocompatibility Complex fusion protein as claimed in claim 2, wherein said extracellular domain comprises residues 5-200 of an MHC Class II alpha chain.

5. A soluble Class II Major Histocompatibility Complex fusion protein as claimed in claim 2, wherein said extracellular domain comprises residues 5-190 of an MHC Class II alpha chain.

6. A soluble Class II Major Histocompatibility Complex fusion protein as claimed in claim 1, wherein said MHC Class II alpha chain is selected from the group consisting of HLA-DR1, HLA-DR2, HLA-DR4, HLA-DQ1, HLA-DQ2 and HLA-DQ8 alpha chains.

7. A soluble Class II Major Histocompatibility Complex fusion protein as claimed in claim 1, wherein said MHC Class II alpha chain is encoded by an HLA allele selected from the group consisting of DRA*0101, DRA*0102, DQA1*0301 and DQA1*0501 alleles.

8. A soluble Class II Major Histocompatibility Complex fusion protein as claimed in claim 1, further comprising an MHC Class II binding peptide, wherein said fusion protein and said MHC Class II binding peptide from a heterodimeric MHC/peptide complex.

9. A soluble Class II Major Histocompatibility Complex fusion protein as claimed in claim 1, wherein said MHC Class II binding domain comprises an extracellular domain of an MHC Class II beta chain.

10. A soluble Class II Major Histocompatibility Complex fusion protein as claimed in claim 9, wherein said extracellular domain comprises residues 5-185 of an MHC Class II beta chain.

11. A soluble Class II Major Histocompatibility Complex fusion protein as claimed in claim 9, wherein said extracellular domain comprises residues 5-205 of an MHC Class II beta chain.

12. A soluble Class II Major Histocompatibility Complex fusion protein as claimed in claim 9, wherein said extracellular domain comprises residues 5-195 of an MHC Class II beta chain.

13. A soluble Class II Major Histocompatibility Complex fusion protein as claimed in claim 1, wherein said MHC Class II beta chain is selected from the group consisting of HLA-DR1, HLA-DR2, HLA-DR4, HLA-DQ1, HLA-DQ2 and HLA-DQ8 beta chains.

14. A soluble Class II Major Histocompatibility Complex fusion protein as claimed in claim 13, wherein said MHC Class II beta chain is encoded by an allele selected from the group consisting of DRB1*01, DRB1*15, DRB1*16, DRB5*01, DQB1*03 and DQB1*02 alleles.

15. A soluble Class II Major Histocompatibility Complex fusion protein as claimed in any one of claims 1-14, wherein said dimerization domain is a leucine zipper domain.

16. A soluble Class II Major Histocompatibility Complex fusion protein as claimed in claim 15, wherein said leucine zipper domain comprises at least four leucine heptads.

17. A soluble Class II Major Histocompatibility Complex fusion protein as claimed in claim 15, wherein said leucine zipper domain is selected from the group consisting of a Fos and a Jun leucine zipper domain.

18. A soluble Class II Major Histocompatibility Complex fusion protein as claimed in any one of claims 1-17, further comprising a first flexible molecular linker interposed between and covalently joining said MHC Class II α chain and said first dimerization domain and a second flexible molecular linker interposed between and covalently joining said MHC Class II ß chain and said second dimerization domain.

19. A soluble Class II Major Histocompatibility Complex fusion protein as claimed in claim 18, wherein said first or second flexible molecular linker comprises a peptide sequence of 1-15 amino acid residues.

20. A soluble Class II Major Histocompatibility Complex fusion protein as claimed in claim 19, wherein said first or second flexible molecular linker comprises a peptide sequence of 5-7 amino acid residues.

21. A soluble Class II Major Histocompatibility Complex fusion protein as claimed in claim 19, wherein a majority of said amino acid residues are selected from the group consisting of alanine, glycine, serine, leucine, isoleucine, and valine residues.

22. A soluble Class II Major Histocompatibility Complex fusion protein as claimed in any one of claims 1-21, further comprising an MHC Class II binding peptide covalently joined to the N-terminus of said MHC Class II α chain, wherein said binding peptide is capable of selectively binding to the MHC Class II molecule including said MHC Class II α chain and said MHC Class II β chain to form an MHC/peptide complex.

23. A soluble Class II Major Histocompatibility Complex fusion protein as claimed in any one of claims 1-21, further comprising an MHC Class II binding peptide covalently joined to the N-terminus of said MHC Class II β chain, wherein said binding peptide is capable of selectively binding to the MHC Class II molecule including said MHC Class II α chain and MHC Class II β chain to form an MHC/peptide complex.

24. An MHC/peptide complex as defined in claims 8, 22 or 23, wherein said MHC Class II molecule is an HLA-DR2 molecule and said binding peptide is selected from the group consisting of residues 85-99, 84-102 and 148-162 of human myelin basic protein.

25. An MHC/peptide complex as defined in claims 8, 22 or 23, wherein said MHC Class II molecule is an HLA-DR4 molecule and said binding peptide is selected from the group consisting of residues 78-93, 97-111, 190-204, 206-220, 251-265, 512-526 and 762-786 of the human desmoglein 3 protein.

26. An MHC/peptide complex as defined in claims 8, 22 or 23, wherein said MHC Class II molecule is an HLA-DQ1 molecule and said binding peptide is selected from the group consisting of residues 78-93, 97-111, 190-204, 206-220, 251-265, 512-526 and 762-786 of the human desmoglein 3 protein.

27. A soluble Class II Major Histocompatibility Complex fusion protein as in any one of claims 22-26, further comprising a flexible molecular linker interposed between and covalently joining said MHC Class II chain and said MHC binding peptide.

28. A soluble Class II Major Histocompatibility Complex fusion protein as claimed in claim 27, wherein said flexible molecular linker comprises a peptide sequence of 10-20 amino acid residues.

29. A soluble Class II Major Histocompatibility Complex fusion protein as claimed in claim 28, wherein said flexible molecular linker comprises a peptide sequence of 12-18 amino acid residues.

30. A soluble Class II Major Histocompatibility Complex fusion protein as claimed in claim 28 or 29, wherein a majority of said amino acid residues are selected from the group consisting of alanine, glycine, serine, leucine, isoleucine, and valine residues.

31. A multimeric Class II Major Histocompatibility Complex fusion protein comprising at least two of.the fusion proteins of any one of claims 1-30.

32. A soluble Class II Major Histocompatibility Complex fusion protein as in any one of claims 1-30, further comprising an N-terminal secretory signal sequence covalently joined to the N-terminus of said fusion protein.

33. A soluble Class II Major Histocompatibility Complex fusion protein as claimed in claim 32, wherein said secretory signal sequence comprises a yeast α-mating factor secretion signal.

34. A soluble Class II Major Histocompatibility Complex fusion protein as claimed in claim 32, wherein said secretory signal sequence comprises a human MHC Class II protein secretion signal.

35. A method for detecting T cells having a defined MHC/peptide complex specificity comprising:
providing monovalent or multivalent MHC/peptide complexes of any one of claims 8, 22 or 23, comprising said defined MHC/peptide complex; and
contacting a population of T cells with said MHC/peptide complex; and
detecting the presence or absence of binding of said MHC/peptide complex and T cells in said population.

36. A method as claimed in claim 35, further comprising isolating T cells reactive with said defined MHC/peptide complex from said population of T cells.

37. A method as claimed in claim 36, wherein said isolation is by means of fluorescence activated cell sorting.

38. An *in vitro* method for stimulating or activating T cells reactive to a defined MHC/peptide complex comprising:
providing a monovalent or multivalent MHC/peptide complex of any one of claims 8, 22 or 23; and
contacting a population of T cells with an immunogenic amount of said MHC/peptide complex.

39. An *in vitro* method for selectively killing T cells reactive to a defined MHC/peptide complex comprising:
providing monovalent or multivalent MHC/peptide complexes of any one of claims 8, 22 or 23, comprising said defined MHC/peptide complex; and
contacting a population of T cells with said MHC/peptide complex, wherein said MHC Class II/peptide complex comprises a cytotoxic substance covalently bound to the fusion protein and capable of killing T cells to which said MHC/peptide complex selectively binds.

40. A soluble monovalent or multivalent MHC/peptide complex as defined in any one of claims 8, 22 or 23, for use in a therapeutic method.

41. A soluble monovalent or multivalent MHC/peptide complex as claimed in claim 40, for conferring to a subject adoptive immunity to a target protein comprising said MHC binding peptide.

42. A soluble monovalent or multivalent MHC/peptide complex as claimed in claim 40, for stimulating or activating T cells reactive to a defined MHC/peptide complex.

43. A soluble monovalent or multivalent MHC/peptide complex as claimed in claim 42, wherein said MHC/peptide complex is syngeneic to said subject.

44. A soluble monovalent or multivalent MHC/peptide complex as claimed in claim 40, for selectively killing T cells reactive to a defined MHC/peptide complex,
wherein said MHC/peptide complex further comprises a covalently bound cytotoxic substance.

45. A soluble monovalent or multivalent MHC/peptide complex as claimed in claim 40, for tolerizing a human subject to a defined MHC Class II binding peptide.

46. A soluble monovalent or multivalent MHC/peptide complex as claimed in claim 45, wherein said soluble Class II MHC fusion protein comprises a Class II MHC binding domain which is syngeneic to said subject.

47. A soluble monovalent or multivalent MHC/peptide complex as claimed in claim 45, wherein said soluble Class II MHC fusion protein comprises a Class II MHC binding domain which is allogeneic to said subject.

48. An isolated nucleic acid encoding a soluble Class II MHC fusion protein of any one of claims 1-34.

49. An isolated nucleic acid encoding an MHC/peptide complex of any one of claims 8, 22, or 23.

50. An MHC/peptide complex as defined in any one of claims 8, 22, or 23, wherein said MHC Class II molecule is an HLA-DR2 molecule and said binding peptide selected from the group consisting of residues 85-99, 84-102 and 148-162 of human myelin basic protein is bound to said MHC Class II fusion protein heterodimer.

51. An MHC/peptide complex as defined in any one of claims 8, 22, or 23, wherein said MHC Class II molecule is an HLA-DR4 molecule; and said binding peptide selected from the group consisting of residues 78-93, 97-111, 190-204, 206-220, 251-265, 512-526 and 762-786 of the human desmoglein 3 protein is bound to said MHC Class II fusion protein heterodimer.

52. An MHC/peptide complex as defined in any one of claims 8, 22, or 23, wherein said MHC Class II molecule is an HLA-DQ1 molecule; and said binding peptide selected from the group consisting of residues 78-93, 97-111, 190-204, 206-220, 251-265, 512-526 and 762-786 of the human desmoglein 3 protein is bound to said MHC Class II fusion protein heterodimer.

## Patentansprüche

1. Lösliches Klasse-II-Haupthistokompatibilitätskomplex-Fusionsprotein umfassend:
ein erstes Polypeptid, umfassend eine Fusion aus, zum N-Terminus hin, mindestens einer MHC-Klasse-II-Bindungsdomäne einer MHC-Klasse-II-α-Kette und, zum C-Terminus hin, einer ersten Coiled-coil-Dimerisierungsdomäne; und
ein zweites Polypeptid, umfassend eine Fusion aus, zum N-Terminus hin, mindestens einer MHC-Klasse-II-Bindungsdomäne einer MHC-Klasse-II-β-Kette und, zum C-Terminus hin, einer zweiten Coiled-coil-Dimerisierungsdomäne;
wobei die ersten und zweiten Polypeptide sich unter physiologischen Bedingungen verbinden und ein heterodimeres MHC-Klasse-II-Molekül bilden, das in der Lage ist, ein MHC-bindendes Peptid zu binden, um einen MHC/Peptid-Komplex bilden.

2. Lösliches Klasse-II-Haupthistokompatibilitätskomplex-Fusionsprotein nach Anspruch 1, wobei die MHC-Klasse-II-Bindungsdomäne eine extrazelluläre Domäne einer MHC-Klasse-II-Alpha-Kette umfaßt.

3. Lösliches Klasse-II-Haupthistokompatibilitätskomplex-Fusionsprotein nach Anspruch 2, wobei die extrazelluläre Domane die Reste 5-180 einer MHC-Klasse-II-Alpha-Kette umfaßt.

4. Lösliches Klasse-II-Haupthistokompatibilitätskomplex-Fusionsprotein nach Anspruch 2, wobei die extrazelluläre Domäne die Reste 5-200 einer MHC-Klasse-II-Alpha-Kette umfaßt.

5. Lösliches Klasse-II-Haupthistokompatibilitätskomplex-Fusionsprotein nach Anspruch 2, wobei die extrazelluläre Domäne die Reste 5-190 einer MHC-Klasse-II-Alpha-Kette umfaßt.

6. Lösliches Klasse-II-Haupthistokompatibilitätskomplex-Fusionsprotein nach Anspruch 1, wobei die MHC-Klasse-II-Alpha-Kette ausgewählt ist aus der Gruppe, bestehend aus HLA-DR1-, HLA-DR2-, HLA-DR4-, HLA-DQ1-, HLA-DQ2- und HLA-DQ8-Alpha-Ketten.

7. Lösliches Klasse-II-Haupthistokompatibilitätskomplex-Fusionsprotein nach Anspruch 1, wobei die MHC-Klasse-II-Alpha-Kette kodiert ist durch ein HLA-Allel, ausgewählt aus der Gruppe, bestehend aus DRA*0101-, DRA*0102-, DQA1*0301- und DQA1*0501-Allelen.

8. Lösliches Klasse-II-Haupthistokompatibilitätskomplex-Fusionsprotein nach Anspruch 1, weiter umfassend ein MHC-Klasse-II-bindendes Peptid, wobei das Fusionsprotein und das MHC-Klasse-II-bindende Peptid einen heterodimeren MHC/Peptid-Komplex bilden.

9. Lösliches Klasse-II-Haupthistokompatibilitätskomplex-Fusionsprotein nach Anspruch 1, wobei die MHC-Klasse-II-Bindungsdomäne eine extrazelluläre Domäne einer MHC-Klasse-II-Beta-Kette umfaßt.

10. Lösliches Kle-sse-II-Haupthistokompatibilitätskomplex-Fusionsprotein nach Anspruch 9, wobei die extrazelluläre Domäne die Reste 5-185 einer MHC-Klasse-ZI-Beta-Kette umfaßt.

11. Lösliches Klasse-II-Haupthistokompatibilitätskomplex-Fusionsprotein nach Anspruch 9, wobei die extrazelluläre Domäne die Reste 5-205 einer MHC-Klasse-II-Beta-Kette umfaßt.

12. Lösliches Klasse-II-Haupthistokompatibilitätskomplex-Fusionsprotein nach Anspruch 9, wobei die extrazelluläre Domäne die Reste 5-195 einer MHC-Klasse-II-Beta-Kette umfaßt.

13. Lösliches Klasse-II-Haupthistokompatibilitätskomplex-Fusionsprotein nach Anspruch 1, wobei die MHC-Klasse-II-Beta-Kette ausgewählt ist aus der Gruppe, bestehend aus HLA-DR1-, HLA-DR2-, HLA-DR4-, HLA-DQ1-, HLA-DQ2- und HLA-DQ8-Beta-Ketten.

14. Lösliches Klasse-II-Haupthistokompatibilitätskomplex-Fusionsprotein nach Anspruch 13, wobei die MHC-Klasse-II-Beta-Kette kodiert ist durch ein Allel, ausgewählt aus der Gruppe, bestehend aus DRB1*01-, DRB1*15-, DRB1*16-, DRB5*01, DQB1*03 und DQB1*02-Allelen.

15. Lösliches Klasse-II-Haupthistokompatibilitätskomplex-Fusionsprotein nach einem der Ansprüche 1-14, wobei die Dimerisierungsdomäne eine Leucin-Zipper-Domäne ist.

16. Lösliches Klasse-II-Haupthistokompatibilitätskomplex-Fusionsprotein nach Anspruch 15, wobei die Leucin-Zipper-Domäne mindestens vier Leucin-Heptaden umfaßt.

17. Lösliches Klasse-II-Haupthistokompatibilitätskomplex-Fusionsprotein nach Anspruch 15, wobei die Leucin-Zipper-Domäne ausgewählt ist aus der Gruppe, bestehend aus einer Fos- und einer Jun-Leucin-Zipper-Domäne.

18. Lösliches Klasse-II-Haupthistokompatibilitätskomplex-Fusionsprotein nach einem der Ansprüche 1-17, ferner umfassend einen ersten flexiblen molekularen Linker, der zwischen der MHC-Klasse-II-α-Kette und der ersten Dimerisierungsdomäne angeordnet ist und diese kovalent verbindet, und einen zweiten flexiblen molekularen Linker, der zwischen der MHC-Klasse-II-β-Kette und der zweiten Dimerisierungsdomäne angeordnet ist und diese kovalent verbindet.

19. Lösliches Klasse-II-Haupthistokompatibilitätskomplex-Fusionsprotein nach Anspruch 18, wobei der erste oder zweite flexible molekulare Linker eine Peptidsequenz von 1-15 Aminosäureresten umfaßt.

20. Lösliches Klasse-II-Haupthistokompatibilitätskomplex-Fusionsprotein nach Anspruch 19, wobei der erste oder zweite flexible molekulare Linker eine Peptidsequenz von 5-7 Aminosäureresten umfaßt.

21. Lösliches Klasse-II-Haupthistokompatibilitätskomplex-Fusionsprotein nach Anspruch 19, wobei eine Mehrheit der Aminosäureresten ausgewählt ist aus der Gruppe, bestehend aus Alanin-, Glycin-, Serin-, Leucin, Isoleucin- und Valin-Resten.

22. Lösliches Klasse-II-Haupthistokompatibilitätskomplex-Fusionsprotein nach einem der Ansprüche 1-21, ferner umfassend ein MHC-Klasse-II-bindendes Peptid, das kovalent an den N-Terminus der MHC-Klasse-II-α-Kette gebunden ist, wobei das bindende Peptid in der Lage ist, selektiv an das MHC-Klasse-II-Molekül einschließlich der MHC-Klasse-II-α-Kette und der MHC-Klasse-II-β-Kette zu binden, um einen MHC/Peptid-Komplex bilden.

23. Lösliches Klasse-II-Haupthistokompatibilitätskomplex-Fusionsprotein nach einem der Ansprüche 1-21, ferner umfassend ein MHC-Klasse-II-bindendes Peptid, das kovalent an den N-Terminus der MHC-Klasse-II-β-Kette gebunden ist, wobei das bindende Peptid in der Lage ist, selektiv an das MHC-Klasse-II-Molekül einschließlich der MHC-Klasse-II-α-Kette und der MHC-Klasse-II-β-Kette zu binden, um einen MHC/Peptid-Komplex zu bilden.

24. MHC/Peptid-Komplex wie in den Ansprüchen 8, 22 oder 23 definiert, wobei das MHC-Klasse-II-Molekül ein HLA-DR2-Molekül ist und das bindende Peptid ausgewählt ist aus der Gruppe, bestehend aus den Resten 85-99, 84-102 und 148-162 eines menschlichen basischen Myelin-Proteins.

25. MHC/Peptid-Komplex wie in den Ansprüchen 8, 22 oder 23 definiert, wobei das MHC-Klasse-II-Molekül ein HLA-DR4-Molekül ist und das bindende Peptid ausgewählt ist aus der Gruppe, bestehend aus den Resten 78-93, 97-111, 190-204, 206-220, 251-265, 512-526 und 762-786 des menschlichen Desmoglein-3-Proteins.

26. MHC/Peptid-Komplex wie in den Ansprüchen 8, 22 oder 23 definiert, wobei das MHC-Klasse-II-Molekül ein HLA-DQ1-Molekül ist und das bindende Peptid ausgewählt ist aus der Gruppe, bestehend aus den Resten 78-93, 97-111, 190-204, 206-220, 251-265, 512-526 und 762-786 des menschlichen Desmoglein-3-Proteins.

27. Lösliches Klasse-II-Haupthistokompatibilitätskomplex-Fusionsprotein nach einem der Ansprüche 22-26, ferner umfassend einen flexiblen molekularen Linker, der zwischen der MHC-Klasse-II-Kette und dem MHC-bindenden Peptid angeordnet ist und diese kovalent verbindet.

28. Lösliches Klasse-II-Haupthistokompatibilitätskomplex-Fusionsprotein nach Anspruch 27, wobei der flexible molekulare Linker eine Peptidsequenz aus 10-20 Aminosäureresten umfaßt.

29. Lösliches Klasse-II-Haupthistokompatibilitätskomplex-Fusionsprotein nach Anspruch 28, wobei der flexible molekulare Linker eine Peptidsequenz aus 12-18 Aminosäureresten umfaßt.

30. Lösliches Klasse-II-Haupthistokompatibilitätskomplex-Fusionsprotein nach Anspruch 28 oder 29, wobei die Mehrheit der Aminosäurereste ausgewählt ist aus der Gruppe, bestehend aus Alanin-, Glycin-, Serin-, Leucin-, Isoleucinund Valinresten.

31. Multimeres Klasse-II-Haupthistokompatibilitätskomplex-Fusionsprotein, umfassend mindestens zwei der Fusionsproteine nach einem der Ansprüche 1-30.

32. Lösliches Klasse-II-Haupthistokompatibilitätskomplex-Fusionsprotein nach einem der Ansprüche 1-30, ferner umfassend eine N-terminale Sekretionssignalsequenz, die kovalent an den N-Terminus des Fusionsproteins gebunden ist.

33. Lösliches Klasse-II-Haupthistokompatibilitätskomplex-Fusionsprotein nach Anspruch 32, wobei die Sekretionssignalsequenz ein Sekretionssignal des Mating-Faktors α aus Hefe umfaßt.

34. Lösliches Klasse-II-Haupthistokompatibilitätskomplex-Fusionsprotein nach Anspruch 32, wobei die Sekretionssignalsequenz ein Sekretionssignal eines menschlichen MHC-Klasse-II-Proteins umfaßt.

35. Verfahren zur Feststellung von T-Zellen, die eine definierte MHC/Peptid-Komplex-Spezifität aufweisen, umfassend:
das Bereitstellen monovalenter oder multivalenter MHC/Peptid-Komplexe nach einem der Ansprüche 8, 22 oder 23, umfassend den definierten MHC/Peptid-Komplex; und
das Inkontaktbringen einer Population von T-Zellen mit dem MHC/Peptid-Komplex; und
das Feststellen der Anwesenheit oder Abwesenheit einer Bindung des MHC/Peptid-Komplexes und der T-Zellen in der Population.

36. Verfahren nach Anspruch 35, ferner umfassend das Isolieren von T-Zellen, die mit dem definierten MHC/Peptid-Komplex aus der Population von T-Zellen reagieren.

37. Verfahren nach Anspruch 36, wobei die Isolierung mittels Durchflußzytometrie erfolgt.

38. In-vitro-Verfahren zur Stimulierung oder Aktivierung von T-Zellen, die mit einem definierten MHC/Peptid-Komplex reagieren, umfassend:
das Bereitstellen eines monovalenten oder multivalenten MHC/Peptid-Komplexes nach einem der Ansprüche 8, 22 oder 23; und
das Inkontaktbringen einer Population von T-Zellen mit einer immunogenen Menge des MHC/Peptid-Komplexes.

39. In-vitro-Verfahren zur selektiven Tötung von T-Zellen, die mit einem definierten MHC/Peptid-Komplex reagieren, umfassend:
das Bereitstellen monovalenter oder multivalenter MHC/Peptid-Komplexe nach einem der Ansprüche 8, 22 oder 23, umfassend den definierten MHC/Peptid-Komplex; und
das Inkontaktbringen einer Population von T-Zellen mit dem MHC/Peptid-Komplex, wobei der MHC-Klasse-II/Peptid-Komplex eine zytotoxische Substanz umfaßt, die kovalent an das Fusionsprotein gebunden und in der Lage ist, T-Zellen zu töten, an die der MHC/Peptid-Komplex selektiv bindet.

40. Löslicher monovalenter oder multivalenter MHC/Peptid-Komplex wie in einem der Ansprüche 8, 22 oder 23 definiert zur Verwendung in einem therapeutischen Verfahren.

41. Löslicher monovalenter oder multivalenter MHC/Peptid-Komplex nach Anspruch 40 zur Vermittlung adoptiver Immunität gegenüber einem Zielprotein an eine Person, umfassend das MHC-bindende Peptid.

42. Löslicher monovalenter oder multivalenter MHC/Peptid-Komplex nach Anspruch 40 zur Stimulierung oder Aktivierung von T-Zellen, die mit einem definierten MHC/Peptid-Komplex reagieren.

43. Löslicher monovalenter oder multivalenter MHC/Peptid-Komplex nach Anspruch 42, wobei der MHC/Peptid-Komplex syngen mit der Person ist.

44. Löslicher monovalenter oder multivalenter MHC/Peptid-Komplex nach Anspruch 40 zur selektiven Tötung von T-Zellen, die mit einem definierten MHC/Peptid-Komplex reagieren, wobei der MHC/Peptid-Komplex ferner eine kovalent gebundene zytotoxische Substanz umfaßt.

45. Löslicher monovalenter oder multivalenter MHC/Peptid-Komplex nach Anspruch 40 zur Tolerisierung eines menschlichen Patienten gegenüber einem definierten MHC-Klasse-II-bindenden Peptid.

46. Löslicher monovalenter oder multivalenter MHC/Peptid-Komplex nach Anspruch 45, wobei das lösliche Klasse-II-MHC-Fusionsprotein eine Klasse-II-MHC-Bindungsdomäne umfaßt, die syngen mit der Person ist.

47. Löslicher monovalenter oder multivalenter MHC/Peptid-Kcmplex nach Anspruch 45, wobei das lösliche Klasse-II-MHC-Fusionsprotein eine Klasse-II-MHC-Bindungsdomäne umfaßt, die allogen zu dem Patienten ist.

48. Isolierte Nukleinsäure, die für ein lösliches Klasse-II-MHC-Fusionsprotein nach einem der Ansprüche 1-34 kodiert.

49. Isolierte Nukleinsäure, die für einen MHC/Peptid-Komplex nach einem der Ansprüche 8, 22 oder 23 kodiert.

50. MHC/Peptid-Komplex wie in einem der Ansprüche 8, 22 oder 23 definiert, wobei das MHC-Klasse-II-Molekül ein HLA-DR2-Molekül ist und das aus der Gruppe, bestehend aus den Resten 85-99, 84-102 und 148-162 des menschlichen basischen Myelin-Proteines, ausgewählte bindende Peptid an das MHC-Klasse-II-Fusionsprotein-Heterodimer gebunden ist.

51. MHC/Peptid-Komplex wie in einem der Ansprüche 8, 22 oder 23 definiert, wobei das MHC-Klasse-II-Molekül ein HLA-DR4-Molekül ist und das aus der Gruppe, bestehend aus den Resten 78-93, 97-111, 190-204, 206-220, 251-265, 512-526 und 762-786 des menschlichen Desmoglein-3-Proteins, ausgewählte bindende Peptid an das MHC-Klasse-II-Fusionsprotein-Heterodimer gebunden ist.

52. MHC/Peptid-Konplex wie in einem der Ansprüche 8, 22 oder 23 definiert, wobei das MHC-Klasse-II-Molekül ein HLA-DQ1-Molekül ist und das aus der Gruppe, bestehend aus den Resten 78-93, 97-111, 190-204, 206-220, 251-265, 512-526 und 762-786 des menschlichen Desmoglein-3-Proteins, ausgewählte bindende Peptid an das MHC-Klasse-II-Fusionsprotein-Heterodimer gebunden ist.

## Revendications

1. Protéine de fusion de complexe majeur d'histocompatibilité de catégorie II soluble, comprenant :
un premier polypeptide comprenant une fusion, vers l'extrémité N-terminale, d'au moins un domaine de liaison de CMH de catégorie II d'une chaîne α de CMH de catégorie II et, vers l'extrémité C-terminale, d'un premier domaine de dimérisation à enroulement en spirale ; et
un second polypeptide comprenant une fusion, vers l'extrémité N-terminale, d'au moins un domaine de liaison de CMH de catégorie II d'une chaîne bêta de CMH de catégorie II et, vers l'extrémité C-terminale, d'un second domaine de dimérisation à enroulement en spirale ;
dans laquelle lesdits premier et second polypeptides s'associent dans des conditions physiologiques pour former une molécule de CMH de catégorie II hétérodimère qui est capable de se lier sélectivement à un peptide de liaison au CMH pour former un complexe CMH/peptide.

2. Protéine de fusion de complexe majeur d'histocompatibilité de catégorie II soluble suivant la revendication 1, dans laquelle ledit domaine de liaison de CMH de catégorie II comprend un domaine extracellulaire d'une chaîne alpha de CMH de catégorie II.

3. Protéine de fusion de complexe majeur d'histocompatibilité de catégorie II soluble suivant la revendication 2, dans laquelle ledit domaine extracellulaire comprend les résidus 5 à 180 d'une chaîne alpha de CMH de catégorie II.

4. Protéine de fusion de complexe majeur d'histocompatibilité de catégorie II soluble suivant la revendication 2, dans laquelle ledit domaine extracellulaire comprend les résidus 5 à 200 d'une chaîne alpha de CMH de catégorie II.

5. Protéine de fusion de complexe majeur d'histocompatibilité de catégorie II soluble suivant la revendication 2, dans laquelle ledit domaine extracellulaire comprend les résidus 5 à 190 d'une chaîne α de CMH de catégorie II.

6. Protéine de fusion de complexe majeur d'histocompatibilité de catégorie II soluble suivant la revendication 1, dans laquelle ladite chaîne alpha de CMH de catégorie II est choisie dans le groupe consistant en les chaînes alpha de HLA-DR1, HLA-DR2, HLA-DR4, HLA-DQ1, HLA-DQ2 et HLA-DQ8.

7. Protéine de fusion de complexe majeur d'histocompatibilité de catégorie II soluble suivant la revendication 1, dans laquelle ladite chaîne alpha de CMH de catégorie II est codée par un allèle de HLA choisi dans le groupe consistant en les allèles DRA*0101, DRA*0102, DQA1*0301 et DQA1*0501.

8. Protéine de fusion de complexe majeur d'histocompatibilité de catégorie II soluble suivant la revendication 1, comprenant en outre un peptide de liaison de CMH de catégorie II, ladite protéine de fusion et ledit peptide de liaison de CMH de catégorie II formant un complexe CMH/peptide hétérodimère.

9. Protéine de fusion de complexe majeur d'histocompatibilité de catégorie II soluble suivant la revendication 1, dans laquelle ledit domaine de liaison de CMH de catégorie II comprend un domaine extracellulaire d'une chaîne bêta de CMH de catégorie II.

10. Protéine de fusion de complexe majeur d'histocompatibilité de catégorie II soluble suivant la revendication 9, dans laquelle ledit domaine extracellulaire comprend les résidus 5 à 185 d'une chaîne bêta de CMH de catégorie II.

11. Protéine de fusion de complexe majeur d'histocompatibilité de catégorie II soluble suivant la revendication 9, dans laquelle ledit domaine extra cellulaire comprend les résidus 5 à 205 d'une chaîne bêta de CMH de catégorie II.

12. Protéine de fusion de complexe majeur d'histocompatibilité de catégorie II soluble suivant la revendication 9, dans laquelle ledit domaine extracellulaire comprend les résidus 5 à 195 d'une chaîne bêta de CMH de catégorie II.

13. Protéine de fusion de complexe majeur d'histocompatibilité de catégorie II soluble suivant la revendication 1, dans laquelle ladite chaîne bêta de CMH de catégorie II est choisie dans le groupe consistant en les chaînes bêta de HLA-DR1, HLA-DR2, HLA-DR4, HLA-DQ1, HLA-DQ2 et HLA-DQ8.

14. Protéine de fusion de complexe majeur d'histocompatibilité de catégorie II soluble suivant la revendication 13, dans laquelle ladite chaîne bêta de CMH de catégorie II est codée par un allèle choisi dans le groupe consistant en les allèles DRB1*01, DRB1*15, DRB1*16, DRB5*01, DQB1*03 et DQB1*02.

15. Protéine de fusion de complexe majeur d'histocompatibilité de catégorie II soluble suivant l'une quelconque des revendications 1 à 14, dans laquelle ledit domaine de dimérisation est un domaine de fermeture-éclair leucine.

16. Protéine de fusion de complexe majeur d'histocompatibilité de catégorie II soluble suivant la revendication 15, dans laquelle ledit domaine de fermeture-éclair leucine comprend au moins quatre heptades de leucine.

17. Protéine de fusion de complexe majeur d'histocompatibilité de catégorie II soluble suivant la revendication 15, dans laquelle ledit domaine de fermeture-éclair leucine est choisi dans le domaine consistant en un domaine de fermeture-éclair leucine Fos et un domaine de fermeture-éclair leucine Jun.

18. Protéine de fusion de complexe majeur d'histocompatibilité de catégorie II soluble suivant l'une quelconque des revendications 1 à 17, comprenant en outre un premier segment de liaison moléculaire flexible intercalé entre, et joignant par covalence, ladite chaîne alpha de CMH de catégorie II et ledit premier domaine de dimérisation, et un second segment de liaison moléculaire flexible intercalé entre, et joignant par covalence, ladite chaîne bêta de CMH de catégorie II et ledit second domaine de dimérisation.

19. Protéine de fusion de complexe majeur d'histocompatibilité de catégorie II soluble suivant la revendication 18, dans laquelle ledit premier ou second segment de liaison moléculaire flexible comprend une séquence peptidique de 1 à 15 résidus d'aminoacides.

20. Protéine de fusion de complexe majeur d'histocompatibilité de catégorie II soluble suivant la revendication 19, dans laquelle ledit premier ou second segment de liaison moléculaire flexible comprend une séquence peptidique de 5 à 7 résidus d'aminoacides.

21. Protéine de fusion de complexe majeur d'histocompatibilité de catégorie II soluble suivant la revendication 19, dans laquelle une proportion dominante desdits résidus d'aminoacides est choisie dans le groupe consistant en les résidus alanine, glycine, sérine, leucine, isoleucine et valine.

22. Protéine de fusion de complexe majeur d'histocompatibilité de catégorie II soluble suivant l'une quelconque des revendications 1 à 21, comprenant en outre un peptide de liaison de CMH de catégorie II joint par covalence à l'extrémité N-terminale de ladite chaîne α de CMH de catégorie II, dans laquelle ledit peptide de liaison est capable de se lier sélectivement à la molécule de CMH de catégorie II comprenant ladite chaîne α de CMH de catégorie II et ladite chaîne β de CMH de catégorie II pour former un complexe CMH/peptide.

23. Protéine de fusion de complexe majeur d'histocompatibilité de catégorie II soluble suivant l'une quelconque des revendications 1 à 21, comprenant en outre un peptide de liaison de CMH de catégorie II joint par covalence à l'extrémité N-terminale de ladite chaîne β de CMH de catégorie II, dans laquelle ledit peptide de liaison est capable de se lier sélectivement à la molécule de CMH de catégorie II comprenant ladite chaîne α de CMH de catégorie II et ladite chaîne β de CMH de catégorie II pour former un complexe CMH/peptide.

24. Complexe CMH/peptide tel que défini dans la revendication 8, 22 ou 23, dans lequel ladite molécule de CMH de catégorie II est une molécule de HLA-DR2 et ledit peptide de liaison est choisi dans le groupe consistant en les résidus 85-99, 84-102 et 148-162 de la protéine basique de myéline humaine.

25. Complexe CMH/peptide tel que défini dans la revendication 8, 22 ou 23, dans lequel ladite molécule de CMH de catégorie II est une molécule de HLA-DR4 et ledit peptide de liaison est choisi dans le groupe consistant en les résidus 78-93, 97-111, 190-204, 206-220, 251-265, 512-526 et 762-786 de la protéine desmogléine 3 humaine.

26. Complexe CMH/peptide tel que défini dans la revendication 8, 22 ou 23, dans lequel ladite molécule de CMH de catégorie II est une molécule de HLA-DQ1 et ledit peptide de liaison est choisi dans le groupe consistant en les résidus 78-93, 97-111, 190-204, 206-220, 251-265, 512-526 et 762-786 de la protéine desmogléine 3 humaine.

27. Protéine de fusion de complexe majeur d'histocompatibilité de catégorie II soluble suivant l'une quelconque des revendications 22 à 26, comprenant en outre un segment de liaison moléculaire flexible intercalé entre, et joignant par covalence, ladite chaîne de CMH de catégorie II et ledit peptide de liaison de CMH.

28. Protéine de fusion de complexe majeur d'histocompatibilité de catégorie II soluble suivant la revendication 27, dans laquelle ledit segment de liaison moléculaire flexible comprend une séquence peptidique de 10 à 20 résidus d'aminoacides.

29. Protéine de fusion de complexe majeur d'histocompatibilité de catégorie II soluble suivant la revendication 28, dans laquelle ledit segment de liaison moléculaire flexible comprend une séquence peptidique de 12 à 18 résidus d'aminoacides.

30. Protéine de fusion de complexe majeur d'histocompatibilité de catégorie II soluble suivant la revendication 28 ou 29, dans laquelle une proportion dominante desdits résidus d'aminoacides est choisie dans le groupe consistant en des résidus alanine, glycine, sérine, leucine, isoleucine et valine.

31. Protéine de fusion de complexe majeur d'histocompatibilité de catégorie II multimère comprenant au moins deux des protéines de fusion suivant l'une quelconque des revendications 1 à 30.

32. Protéine de fusion de complexe majeur d'histocompatibilité de catégorie II soluble suivant l'une quelconque des revendications 1 à 30, comprenant en outre une séquence signal de sécrétion N-terminale jointe par covalence à l'extrémité N-terminale de ladite protéine de fusion.

33. Protéine de fusion de complexe majeur d'histocompatibilité de catégorie II soluble suivant la revendication 32, dans laquelle ladite séquence signal de sécrétion comprend un signal de sécrétion de facteur d'appariement α de levure.

34. Protéine de fusion de complexe majeur d'histocompatibilité de catégorie II soluble suivant la revendication 32, dans laquelle ladite séquence signal de sécrétion comprend un signal de sécrétion de protéine de catégorie II de CMH humain.

35. Méthode pour détecter des lymphocytes T présentant une spécificité pour un complexe CMH/peptide défini, comprenant les étapes consistant :
à fournir des complexes CMH/peptide monovalents ou multivalents suivant l'une quelconque des revendications 8, 22 et 23, comprenant ledit complexe CMH/peptide défini ; et
mettre en contact une population de lymphocytes T avec ledit complexe CMH/peptide ; et
à détecter la présence ou l'absence de liaison dudit complexe CMH/peptide et des lymphocytes T dans ladite population.

36. Méthode suivant la revendication 35, comprenant en outre l'isolement de lymphocytes T réactifs avec ledit complexe CMH/peptide défini à partir de ladite population de lymphocytes T.

37. Méthode suivant la revendication 36, dans laquelle ledit isolement est effectué par tri cellulaire activé par fluorescence.

38. Méthode in vitro pour stimuler ou activer des lymphocytes T réactifs vis-à-vis d'un complexe CMH/peptide défini, comprenant les étapes consistant :
à fournir un complexe CMH/peptide monovalent ou multivalent suivant l'une quelconque des revendications 8, 22 et 23 ; et
à mettre en contact une population de lymphocytes T avec une quantité immunogène dudit complexe CMH/peptide.

39. Méthode in vitro pour tuer sélectivement des lymphocytes T réactifs vis-à-vis d'un complexe CMH/peptide défini, comprenant les étapes consistant :
à fournir des complexes CMH/peptide monovalents ou multivalents suivant l'une quelconque des revendications 8, 22 et 23, comprenant ledit complexe CMH/peptide défini ; et
à mettre en contact une population de lymphocytes T avec ledit complexe CMH/peptide, ledit complexe CMH de catégorie II/peptide comprenant une substance cytotoxique liée par covalence à la protéine de fusion et capable de tuer les lymphocytes T auxquels ledit complexe CMH/peptide se lie sélectivement.

40. Complexe CMH/peptide monovalent ou multivalent soluble tel que défini dans l'une quelconque des revendications 8, 22 et 23, destiné à être utilisé dans une méthode thérapeutique.

41. Complexe CMH/peptide monovalent ou multivalent soluble suivant la revendication 40, destiné à conférer à un sujet une immunité adoptive à une protéine cible comprenant ledit peptide de liaison de CMH.

42. Complexe CMH/peptide monovalent ou multivalent soluble suivant la revendication 40, destiné à stimuler ou activer des lymphocytes T réactifs vis-à-vis d'un complexe CMH/peptide défini.

43. Complexe CMH/peptide monovalent ou multivalent soluble suivant la revendication 42, ledit complexe CMH/peptide étant syngénique vis-à-vis dudit sujet.

44. Complexe CMH/peptide monovalent ou multivalent soluble suivant la revendication 40, destiné à tuer sélectivement des lymphocytes T réactifs avec un complexe CMH/peptide défini, ledit complexe CMH/peptide comprenant en outre une substance cytotoxique liée par covalence.

45. Complexe CMH/peptide monovalent ou multivalent soluble suivant la revendication 40, destiné à rendre tolérant un sujet humain vis-à-vis d'un peptide de liaison de CMH de catégorie II défini.

46. Complexe CMH/peptide monovalent ou multivalent soluble suivant la revendication 45, dans lequel ladite protéine de fusion de CMH de catégorie II soluble comprend un domaine de liaison de CMH de catégorie II qui est syngénique vis-à-vis dudit sujet.

47. Complexe CMH/peptide monovalent ou multivalent soluble suivant la revendication 45, dans lequel ladite protéine de fusion de CMH de catégorie II soluble comprend un domaine de liaison de CMH de catégorie II qui est allogénique vis-à-vis dudit sujet.

48. Acide nucléique isolé codant pour une protéine de fusion de CMH de catégorie II soluble suivant l'une quelconque des revendications 1 à 34.

49. Acide nucléique isolé codant pour un complexe CMH/peptide suivant l'une quelconque des revendications 8, 22 et 23.

50. Complexe CMH/peptide tel que défini dans l'une quelconque des revendications 8, 22 et 23, dans lequel ladite molécule de CMH de catégorie II est une molécule de HLA-DR2 et ledit peptide de liaison choisi dans le groupe consistant en les résidus 85-99, 84-102 et 148-162 de la protéine basique de myéline humaine est lié audit hétérodimère de protéine de fusion de CMH de catégorie II.

51. Complexe CMH/peptide tel que défini dans l'une quelconque des revendications 8, 22 et 23, dans lequel ladite molécule de CMH de catégorie II est une molécule de HLA-DR4, et ledit peptide de liaison choisi dans le groupe consistant en les résidus 78-93, 97-111, 190-204, 206-220, 251-265, 512-526 et 762-786 de la protéine desmogléine 3 humaine est lié audit hétérodimère de protéine de fusion de CMH de catégorie II.

52. Complexe CMH/peptide tel que défini dans l'une quelconque des revendications 8, 22 et 23, dans lequel ladite molécule de CMH de catégorie II est une molécule de HLA-DQ1, et ledit peptide de liaison choisi dans le groupe consistant en les résidus 78-93, 97-111, 190-204, 206-220, 251-265, 512-526 et 762-786 de la protéine desmogléine 3 humaine est lié audit hétérodimère de protéine de fusion de CMH de catégorie II.


### SEQUENCE LISTING

(1) GENERAL INFORMATION:
   (i) APPLICANT: WUCHERPFENNIG, Kai W STROMINGER, Jack L President and Fellows of Harvard College
   (ii) TITLE OF INVENTION: SOLUBLE MONOVALENT AND MULTIVALENT MHC CLASSII FUSION PROTEINS AND USES THEREFOR
   (iii) NUMBER OF SEQUENCES: 10
   (iv) CORRESPONDENCE ADDRESS:
      (A) ADDRESSEE: TESTA, HURWITZ & THIBEAULT, LLP
      (B) STREET: 125 HIGH STREET
      (C) CITY: BOSTON
      (D) STATE: MA
      (E) COUNTRY: USA
      (F) ZIP: 02110
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 60/024,077
      (B) FILING DATE: 16-AUG-1996
   (viii) ATTORNEY/AGENT INFORMATION:
      (A) NAME: Twomey, Michael J
      (B) REGISTRATION NUMBER: 38,349
      (C) REFERENCE/DOCKET NUMBER: HAR-002PR
   (ix) TELECOMMUNICATION INFORMATION:
      (A) TELEPHONE: (617) 248 7000
      (B) TELEFAX: (617) 248 7100
(2) INFORMATION FOR SEQ ID NO:1:
   (i) SEQUENCE CHARACTERISTICS:
      (A) LENGTH: 750 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..735
   (ix) FEATURE:
      (A) NAME/KEY: misc feature
      (B) LOCATION: 1..2T
      (D) OTHER INFORMATION: /note= "3' end of secretory signal"
   (ix) FEATURE:
      (A) NAME/KEY: misc feature
      (B) LOCATION: 22..594
      (D) OTHER INFORMATION: /note= "DRA*0101 extracellular domain"
   (ix) FEATURE:
      (A) NAME/KEY: misc_feature
      (B) LOCATION: 595..615
      (D) OTHER INFORMATION: /note= "Linker sequence"
   (ix) FEATURE:
      (A) NAME/KEY: misc feature
      (B) LOCATION: 616..735
      (D) OTHER INFORMATION: /note= "Fos leucine zipper domain"
   (xi) SEQUENCE DESCRIPTION: SEQ ID NO:1:
   GTA TCT CTC GAG AAA AGA GAG ATC AAA GAA GAA CAT GTG ATC ATC CAG      48
   Val Ser Leu Glu Lys Arg Glu Ile Lys Glu Glu His Val Ile Ile Gln
    1               5                  10                  15
   GCC GAG TTC TAT CTG AAT CCT GAC CAA TCA GGC GAG TTT ATG TTT GAC      96
   Ala Glu Phe Tyr Leu Asn Pro Asp Gln Ser Gly Glu Phe Met Phe Asp
               20                  25                  30
   TTT GAT GGT GAT GAG ATT TTC CAT GTG GAT ATG GCA AAG AAG GAG ACG     144
   Phe Asp Gly Asp Glu Ile Phe His Val Asp Met Ala Lys Lys Glu Thr
           35                  40                  45
   GTC TGG CGG CTT GAA GAA TTT GGA CGA TTT GCC AGC TTT GAG GCT CAA     192
   Val Trp Arg Leu Glu Glu Phe Gly Arg Phe Ala Ser Phe Glu Ala Gln
       50                  55                  60
   GGT GCA TTG GCC AAC ATA GCT GTG GAC AAA GCC AAC TTG GAA ATC ATG     240
   Gly Ala Leu Ala Asn Ile Ala Val Asp Lys Ala Asn Leu Glu Ile Met
    65                  70                  75                  80
   ACA AAG CGC TCC AAC TAT ACT CCG ATC ACC AAT GTA CCT CCA GAG GTA     288
   Thr Lys Arg Ser Asn Tyr Thr Pro Ile Thr Asn Val Pro Pro Glu Val
                   85                  90                  95
   ACT GTG CTC ACG AAC AGC CCT GTG GAA CTG AGA GAG CCC AAC GTC CTC     336
   Thr Val Leu Thr Asn Ser Pro Val Glu Leu Arg Glu Pro Asn Val Leu
               100                 105                 110
   ATC TGT TTC ATA GAC AAG TTC ACC CCA CCA GTG GTC AAT GTC ACG TGG     384
   Ile Cys Phe Ile Asp Lys Phe Thr Pro Pro Val Val Asn Val Thr Trp
           115                 120                 125
   CTT CGA AAT GGA AAA CCT GTC ACC ACA GGA GTG TCA GAG ACA GTC TTC     432
   Leu Arg Asn Gly Lys Pro Val Thr Thr Gly Val Ser Glu Thr Val Phe
       130                 135                 140
   CTG CCC AGG GAA GAC CAC CTT TTC CGC AAG TTC CAC TAT CTC CCC TTC     480
   Leu Pro Arg Glu Asp His Leu Phe Arg Lys Phe His Tyr Leu Pro Phe
   145                 150                 155                 160
   CTG CCC TCA ACT GAG GAC GTT TAC GAC TGC AGG GTG GAG CAC TGG GGC     528
   Leu Pro Ser Thr Glu Asp Val Tyr Asp Cys Arg Val Glu His Trp Gly
                   165                 170                 175
   TTG GAT GAG CCT CTT CTC AAG CAC TGG GAG TTT GAT GCT CCA AGC CCT     576
   Leu Asp Glu Pro Leu Leu Lys His Trp Glu Phe Asp Ala Pro Ser Pro
               180                 185                 190
   CTC CCA GAG ACT ACA GAG GTC GAC GGA GGT GGC GGC GGT TTA ACT GAT     624
   Leu Pro Glu Thr Thr Glu Val Asp Gly Gly Gly Gly Gly Leu Thr Asp
           195                 200                 205
   ACA CTC CAA GCG GAG ACA GAT CAA CTT GAA GAC GAG AAG TCT GCG TTG     672
   Thr Leu Gln Ala Glu Thr Asp Gln Leu Glu Asp Glu Lys Ser Ala Leu
       210                 215                 220
   CAG ACC GAG ATT GCC AAT CTA CTG AAA GAG AAG GAA AAA CTG GAG TTC     720
   Gln Thr Glu Ile Ala Asn Leu Leu Lys Glu Lys Glu Lys Leu Glu Phe
   225                 230                 235                 240
   ATC CTG GCC GCC CAT TGAGAATTCT ATGAC                                750
   Ile Leu Ala Ala His
                   245
(2) INFORMATION FOR SEQ ID NO:2:
   (i) SEQUENCE CHARACTERISTICS:
      (A) LENGTH: 245 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Val Ser Leu Glu Lys Arg Glu Ile Lys Glu Glu His Val Ile Ile Gln
    1               5                  10                  15
   Ala Glu Phe Tyr Leu Asn Pro Asp Gln Ser Gly Glu Phe Met Phe Asp
               20                  25                  30
   Phe Asp Gly Asp Glu Ile Phe His Val Asp Met Ala Lys Lys Glu Thr
           35                  40                  45
   Val Trp Arg Leu Glu Glu Phe Gly Arg Phe Ala Ser Phe Glu Ala Gln
       50                  55                  60
   Gly Ala Leu Ala Asn Ile Ala Val Asp Lys Ala Asn Leu Glu Ile Met
   65                  70                  75                  80
   Thr Lys Arg Ser Asn Tyr Thr Pro Ile Thr Asn Val Pro Pro Glu Val
                   85                  90                  95
   Thr Val Leu Thr Asn Ser Pro Val Glu Leu Arg Glu Pro Asn Val Leu
               100                 105                 110
   Ile Cys Phe Ile Asp Lys Phe Thr Pro Pro Val Val Asn Val Thr Trp
           115                 120                 125
   Leu Arg Asn Gly Lys Pro Val Thr Thr Gly Val Ser Glu Thr Val Phe
       130                 135                 140
   Leu Pro Arg Glu Asp His Leu Phe Arg Lys Phe His Tyr Leu Pro Phe
   145                 150                 155                 160
   Leu Pro Ser Thr Glu Asp Val Tyr Asp Cys Arg Val Glu His Trp Gly
                   165                 170                 175
   Leu Asp Glu Pro Leu Leu Lys His Trp Glu Phe Asp Ala Pro Ser Pro
               180                 185                 190
   Leu Pro Glu Thr Thr Glu Val Asp Gly Gly Gly Gly Gly Leu Thr Asp
           195                 200                 205
   Thr Leu Gln Ala Glu Thr Asp Gln Leu Glu Asp Glu Lys Ser Ala Leu
       210                 215                 220
   Gln Thr Glu Ile Ala Asn Leu Leu Lys Glu Lys Glu Lys Leu Glu Phe
   225                 230                 235                 240
   Ile Leu Ala Ala His
                   245
(2) INFORMATION FOR SEQ ID NO:3:
   (i) SEQUENCE CHARACTERISTICS:
      (A) LENGTH: 771 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..756
   (ix) FEATURE:
      (A) NAME/KEY: misc feature
      (B) LOCATION: 1..2T
      (D) OTHER INFORMATION: /note= "3' end of secretory signal"
   (ix) FEATURE:
      (A) NAME/KEY: misc feature
      (B) LOCATION: 22..615
      (D) OTHER INFORMATION: /note= "DRB1*1501 extracellular domain"
   (ix) FEATURE:
      (A) NAME/KEY: misc_feature
      (B) LOCATION: 616.-636
      (D) OTHER INFORMATION: /note= "Linker sequence"
   (ix) FEATURE:
      (A) NAME/KEY: misc feature
      (B) LOCATION: 637..756
      (D) OTHER INFORMATION: /note= "Jun leucine zipper domain"
   (xi) SEQUENCE DESCRIPTION: SEQ ID NO:3:
   GTA TCT CTC GAG AAA AGA GAG GGG GAC ACC CGA CCA CGT TTC CTG TGG      48
   Val Ser Leu Glu Lys Arg Glu Gly Asp Thr Arg Pro Arg Phe Leu Trp
    1               5                  10                  15
   CAG CCT AAG AGG GAG TGT CAT TTC TTC AAT GGG ACG GAG CGG GTG CGG      96
   Gln Pro Lys Arg Glu Cys His Phe Phe Asn Gly Thr Glu Arg Val Arg
                20                  25                  30
   TTC CTG GAC AGA TAC TTC TAT AAC CAG GAG GAG TCC GTG CGC TTC GAC     144
   Phe Leu Asp Arg Tyr Phe Tyr Asn Gln Glu Glu Ser Val Arg Phe Asp
            35                  40                  45
   AGC GAC GTG GGG GAG TTC CGG GCG GTG ACG GAG CTG GGG CGG CCT GAC     192
   Ser Asp Val Gly Glu Phe Arg Ala Val Thr Glu Leu Gly Arg Pro Asp
        50                  55                  60
   GCT GAG TAC TGG AAC AGC CAG AAG GAC ATC CTG GAG CAG GCG CGG GCC     240
   Ala Glu Tyr Trp Asn Ser Gln Lys Asp Ile Leu Glu Gln Ala Arg Ala
    65                  70                  75                  80
   GCG GTG GAC ACC TAC TGC AGA CAC AAC TAC GGG GTT GTG GAG AGC TTC     288
   Ala Val Asp Thr Tyr Cys Arg His Asn Tyr Gly Val Val Glu Ser Phe
                    85                  90                  95
   ACA GTG CAG CGG CGA GTC CAA CCT AAG GTG ACT GTA TAT CCT TCA AAG     336
   Thr Val Gln Arg Arg Val Gln Pro Lys Val Thr Val Tyr Pro Ser Lys
               100                 105                 110
   ACC CAG CCC CTG CAG CAC CAC AAC CTC CTG GTC TGC TCT GTG AGT GGT     384
   Thr Gln Pro Leu Gln His His Asn Leu Leu Val Cys Ser Val Ser Gly
           115                 120                 125
   TTC TAT CCA GGC AGC ATT GAA GTC AGG TGG TTC CTG AAC GGC CAG GAA     432
   Phe Tyr Pro Gly Ser Ile Glu Val Arg Trp Phe Leu Asn Gly Gln Glu
       130                 135                 140
   GAG AAG GCT GGG ATG GTG TCC ACA GGC CTG ATC CAG AAT GGA GAC TGG     480
   Glu Lys Ala Gly Met Val Ser Thr Gly Leu Ile Gln Asn Gly Asp Trp
   145                 150                 155                 160
   ACC TTC CAG ACC CTG GTG ATG CTG GAA ACA GTT CCT CGA AGT GGA GAG     528
   Thr Phe Gln Thr Leu Val Met Leu Glu Thr Val Pro Arg Ser Gly Glu
                   165                 170                 175
   GTT TAC ACC TGC CAA GTG GAG CAC CCA AGC GTG ACA AGC CCT CTC ACA     576
   Val Tyr Thr Cys Gln Val Glu His Pro Ser Val Thr Ser Pro Leu Thr
               180                 185                 190
   GTG GAA TGG AGA GCA CGG TCT GAA TCT GCA CAG AGC AAG GTC GAC GGA     624
   Val Glu Trp Arg Ala Arg Ser Glu Ser Ala Gln Ser Lys Val Asp Gly
           195                 200                 205
   GGT GGC GGC GGT CGC ATC GCC CGG CTC GAG GAA AAA GTG AAA ACC TTG     672
   Gly Gly Gly Gly Arg Ile Ala Arg Leu Glu Glu Lys Val Lys Thr Leu
       210                 215                 220
   AAA GCT CAG AAC TCG GAG CTC GCG TCC ACG GCC AAC ATG CTC AGG GAA     720
   Lys Ala Gln Asn Ser Glu Leu Ala Ser Thr Ala Asn Met Leu Arg Glu
   225                 230                 235                 240
   CAG GTG GCA CAG CTT AAA CAG AAA GTC ATG AAC CAT TGAGAATTCT          766
   Gln Val Ala Gln Leu Lys Gln Lys Val Met Asn His
                   245                 250
   ATGAC                                                               771
(2) INFORMATION FOR SEQ ID NO:4:
   (i) SEQUENCE CHARACTERISTICS:
      (A) LENGTH: 252 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Val Ser Leu Glu Lys Arg Glu Gly Asp Thr Arg Pro Arg Phe Leu Trp
   1               5                   10                  15
   Gln Pro Lys Arg Glu Cys His Phe Phe Asn Gly Thr Glu Arg Val Arg
               20                  25                  30
   Phe Leu Asp Arg Tyr Phe Tyr Asn Gln Glu Glu Ser Val Arg Phe Asp
           35                  40                  45
   Ser Asp Val Gly Glu Phe Arg Ala Val Thr Glu Leu Gly Arg Pro Asp
       50                  55                  60
   Ala Glu Tyr Trp Asn Ser Gln Lys Asp Ile Leu Glu Gln Ala Arg Ala
   65                  70                  75                  80
   Ala Val Asp Thr Tyr Cys Arg His Asn Tyr Gly Val Val Glu Ser Phe
                   85                  90                  95
   Thr Val Gln Arg Arg Val Gln Pro Lys Val Thr Val Tyr Pro Ser Lys
               100                 105                 110
   Thr Gln Pro Leu Gln His His Asn Leu Leu Val Cys Ser Val Ser Gly
           115                 120                 125
   Phe Tyr Pro Gly Ser Ile Glu Val Arg Trp Phe Leu Asn Gly Gln Glu
       130                 135                 140
   Glu Lys Ala Gly Met Val Ser Thr Gly Leu Ile Gln Asn Gly Asp Trp
   145                 150                 155                 160
   Thr Phe Gln Thr Leu Val Met Leu Glu Thr Val Pro Arg Ser Gly Glu
                   165                 170                 175
   Val Tyr Thr Cys Gln Val Glu His Pro Ser Val Thr Ser Pro Leu Thr
               180                 185                 190
   Val Glu Trp Arg Ala Arg Ser Glu Ser Ala Gln Ser Lys Val Asp Gly
           195                 200                 205
   Gly Gly Gly Gly Arg Ile Ala Arg Leu Glu Glu Lys Val Lys Thr Leu
       210                 215                 220
   Lys Ala Gln Asn Ser Glu Leu Ala Ser Thr Ala Asn Met Leu Arg Glu
   225                 230                 235                 240
   Gln Val Ala Gln Leu Lys Gln Lys Val Met Asn His
                   245                 250
(2) INFORMATION FOR SEQ ID NO:5:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ix) FEATURE:
      (A) NAME/KEY: misc feature
      (B) LOCATION: 1..42
      (D) OTHER INFORMATION: /note= "synthetic, PCR primer"
   (xi) SEQUENCE DESCRIPTION: SEQ ID NO:5:
   GTATCTCTCG AGAAAAGAGA GATCAAAGAA GAACATGTGA TC 42
(2) INFORMATION FOR SEQ ID NO:6:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ix) FEATURE:
      (A) NAME/KEY: misc feature
      (B) LOCATION: 1..39
      (D) OTHER INFORMATION: /note= "synthetic, PCR primer"
   (xi) SEQUENCE DESCRIPTION: SEQ ID NO:6:
   GTCATAGAAT TCTCAATGGG CGGCCAGGAT GAACTCCAG 39
(2) INFORMATION FOR SEQ ID NO:7:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ix) FEATURE:
      (A) NAME/KEY: misc feature
      (B) LOCATION: 1..42
      (D) OTHER INFORMATION: /note= "synthetic, PCR primer"
   (xi) SEQUENCE DESCRIPTION: SEQ ID NO:7:
   GTATCTCTCG AGAAAAGAGA GGGGGACACC CGACCACGTT TC 42
(2) INFORMATION FOR SEQ ID NO:8:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ix) FEATURE:
      (A) NAME/KEY: misc feature
      (B) LOCATION: 1..39
      (D) OTHER INFORMATION: /note= "synthetic, PCR primer"
   (xi) SEQUENCE DESCRIPTION: SEQ ID NO:8:
   GTCATAGAAT TCTCAATGGT TCATGACTTT CTGTTTAAG 39
(2) INFORMATION FOR SEQ ID NO:9:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ix) FEATURE:
      (A) NAME/KEY: misc feature
      (B) LOCATION: 1..14
      (D) OTHER INFORMATION: /note= "synthetic, biotin ligase recognition sequence"
   (xi) SEQUENCE DESCRIPTION: SEQ ID NO:9:
   Leu Gly Gly Ile Phe Glu Ala Met Lys Met Glu Leu Arg Asp
   1 5 10
(2) INFORMATION FOR SEQ ID NO:10:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ix) FEATURE:
      (A) NAME/KEY: misc feature
      (B) LOCATION: 1..16
      (D) OTHER INFORMATION: /note= "synthetic, linker sequence"
   (xi) SEQUENCE DESCRIPTION: SEQ ID NO:10:
   Ser Gly Gly Gly Ser Leu Val Pro Arg Gly Ser Gly Gly Gly Gly Ser
   1 5 10 15